(19) Europäisches Patentamt
European Patent Office
Office européen des brevets

(11) EP 3 429 349 B1

(12) **FASCICULE DE BREVET EUROPEEN**

(45) Date de publication et mention de la délivrance du brevet:
**25.11.2020 Bulletin 2020/48**

(21) Numéro de dépôt: **17710580.6**

(22) Date de dépôt: **17.03.2017**

(51) Int Cl.:
*A01N 25/30* (2006.01) *A01N 25/02* (2006.01)
*A61Q 19/00* (2006.01) *A61K 8/86* (2006.01)
*A61K 8/39* (2006.01) *A01N 43/16* (2006.01)
*A01N 35/02* (2006.01) *A01N 31/16* (2006.01)
*A01N 25/04* (2006.01) *C11D 1/74* (2006.01)

(86) Numéro de dépôt international:
**PCT/EP2017/056465**

(87) Numéro de publication internationale:
**WO 2017/158194 (21.09.2017 Gazette 2017/38)**

(54) **CONCENTRE COMPRENANT UN MEL ET UN ESTER D'ACIDE GRAS ET DE POLYETHYLENE GLYCOL DE HLB SUPERIEUR OU EGAL A 12**

KONZENTRAT MIT EINEM MEL UND EINEM FETTSÄUREESTER UND POLYETHYLENGLYKOL MIT EINEM HLB-WERT GRÖSSER ODER GLEICH 12

CONCENTRATE COMPRISING A MEL AND AN ESTER OF FATTY ACID AND POLYETHYLENE GLYCOL HAVING AN HLB VALUE GREATER THAN OR EQUAL TO 12

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.03.2016 FR 1652288**

(43) Date de publication de la demande:
**23.01.2019 Bulletin 2019/04**

(73) Titulaire: **Oleon N.V.**
**9940 Evergem (Ertvelde) (BE)**

(72) Inventeurs:
- **RANDU, Marion**
**60600 Clermont (FR)**
- **HERY, Sylvie**
**60880 Jaux (FR)**
- **RAVIER, Pierre**
**60200 Compiegne (FR)**
- **DEPREY, Sophie**
**60280 Margny-Les-Compiegne (FR)**

(74) Mandataire: **Santarelli**
**49, avenue des Champs-Elysées**
**75008 Paris (FR)**

(56) Documents cités:
**EP-A1- 1 964 546 US-A1- 2013 142 855**

- **DATABASE WPI Week 201116 Thomson Scientific, London, GB; AN 2011-B53703 XP002760455, -& JP 2011 026278 A (TOYOBO KK) 10 février 2011 (2011-02-10)**
- **DATABASE WPI Week 200952 Thomson Scientific, London, GB; AN 2009-L99012 XP002760456, -& JP 2009 167158 A (TOYOBO KK) 30 juillet 2009 (2009-07-30)**
- **DATABASE WPI Week 200951 Thomson Scientific, London, GB; AN 2009-L99013 XP002760457, -& JP 2009 167157 A (TOYOBO KK) 30 juillet 2009 (2009-07-30)**
- **DATABASE WPI Week 201010 Thomson Scientific, London, GB; AN 2010-B09879 XP002760458, -& JP 2010 018560 A (TOYOBO KK) 28 janvier 2010 (2010-01-28)**
- **DATABASE WPI Week 201116 Thomson Scientific, London, GB; AN 2011-B53710 XP002760459, -& JP 2011 026276 A (TOYOBO KK) 10 février 2011 (2011-02-10)**
- **TOKUMA FUKUOKA ET AL: "Application of Yeast Glycolipid Biosurfactant, Mannosylerythritol Lipid, as Agrospreaders", JOURNAL OF OLEO SCIENCE, vol. 64, no. 6, 1 janvier 2015 (2015-01-01), pages 689-695, XP055280365, JP ISSN: 1345-8957, DOI: 10.5650/jos.ess15017**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 429 349 B1

- **KITAMOTO D ET AL: "MICROBIAL CONVERSION OF N-ALKANES INTO GLYCOLIPID BIOSURFACTANTS, MANNOSYLERYTHRITOL LIPIDS, BY PSEUDOZYMA (CANDIDA ANTARCTICA)", BIOTECHNOLOGY LETTERS, SPRINGER NETHERLANDS, NL, vol. 23, no. 20, 1 octobre 2001 (2001-10-01), pages 1709-1714, XP008014848, ISSN: 0141-5492, DOI: 10.1023/A:1012464717259**
- **JOSEPH IRUDAYARAJ ARUTCHELVI ET AL: "Mannosylerythritol lipids: a review", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 35, no. 12, 21 août 2008 (2008-08-21) , pages 1559-1570, XP019637554, ISSN: 1476-5535, DOI: 10.1007/S10295-008-0460-4**
- **MNIF INÈS ET AL: "Glycolipid biosurfactants: Potential related biomedical and biotechnological applications", CARBOHYDRATE RESEARCH, vol. 416, 1 octobre 2015 (2015-10-01), pages 59-69, XP055371162, GB ISSN: 0008-6215, DOI: 10.1016/j.carres.2015.07.016**
- **N. LOURITH ET AL: "Natural surfactants used in cosmetics: glycolipids", INTERNATIONAL JOURNAL OF COSMETIC SCIENCE., vol. 31, no. 4, 1 août 2009 (2009-08-01), pages 255-261, XP055371172, NL ISSN: 0142-5463, DOI: 10.1111/j.1468-2494.2009.00493.x**
- **Anonymous: "PEG esters | Echem", , 1 mars 2014 (2014-03-01), XP055371185, Extrait de l'Internet: URL:http://web.archive.org/web/20140301052052/http://www.echem-group.com/product/peg-esters [extrait le 2017-05-10]**
- **Anonymous: "POLYETHYLENEGLYCOL MONOSTEARATE", , 20 mai 2006 (2006-05-20), XP055371188, Extrait de l'Internet: URL:http://www.chemicalland21.com/specialtychem/perchem/POLYETHYLENEGLYCOL MONOSTEARATE.htm [extrait le 2017-05-10]**

## Description

La présente invention se rapporte à un concentré, à des compositions le comprenant, et à une émulsion le comprenant. La présente invention concerne également un procédé de préparation du concentré, des compositions, et de l'émulsion selon l'invention, et l'utilisation du concentré, en particulier en tant qu'agent tensioactif, et plus particulièrement en tant qu'agent émulsifiant.

Les agents tensioactifs trouvent une application dans de nombreux domaines. Il est notamment connu d'utiliser des agents tensioactifs, dans l'industrie du nettoyage, par exemple dans la préparation de produits d'entretien ou de nettoyage ménager, ou encore dans l'industrie cosmétique. L'utilisation d'agents tensioactifs présente également un intérêt particulier dans l'industrie agricole, notamment pour la protection des plantes. Dans celle-ci, les agents tensioactifs, et plus particulièrement les agents émulsifiants, sont généralement utilisés dans la préparation de produits phytosanitaires, telles que des bouillies phytosanitaires, comme par exemple des herbicides, fongicides, insecticides, algicides ou encore des produits de stimulation des défenses des plantes. Ces produits phytosanitaires se présentent souvent sous la forme d'émulsion afin de faire co-exister ensemble un solvant préféré, à savoir l'eau, avec des actifs généralement hydrophobes. Dans ce cas, le ou les tensioactif(s) présent(s) dans ces produits présente(nt) également une propriété émulsifiante.

Outre leurs propriétés tensioactives et éventuellement émulsifiantes, ces agents peuvent posséder d'autres propriétés. A titre d'exemple, dans la cadre d'une utilisation phytosanitaire, un tel agent peut également posséder une propriété pénétrante, humectante et/ou mouillante. Un agent tensioactif, et plus particulièrement émulsifiant, présentant une propriété mouillante favorise l'étalement et la rétention de l'émulsion le comprenant sur les cultures. Il en résulte, d'une part, une réduction de la quantité d'émulsion phytosanitaire à pulvériser sur les cultures, et d'autre part, une amélioration de l'efficacité des émulsions phytosanitaires. L'utilisation d'agents tensioactifs ayant des propriétés émulsifiantes et mouillantes permet ainsi une réduction des efforts et des coûts associés au traitement des cultures.

En outre, quels que soient les types d'industrie dans lesquels ils sont utilisés, il est préférable que les agents tensioactifs soient respectueux de l'environnement et de moindre toxicité pour les opérateurs. Cela est particulièrement important lorsqu'ils sont utilisés dans la préparation d'émulsions phytosanitaires, car celles-ci sont généralement déversées en quantité non négligeable sur les cultures, de sorte qu'il est préférable, voire nécessaire, que les agents tensioactifs soient écologiquement avantageux, et notamment biodégradables.

Plus particulièrement, il serait intéressant de développer des agent tensioactifs :

- possédant une excellente propriété tensioactive, et plus particulièrement émulsifiante,
- permettant l'obtention d'émulsions stables,
- possédant une bonne capacité à augmenter le pouvoir mouillant des émulsions les comprenant, telles que des émulsions phytosanitaires, et
- qui seraient en outre respectueux de l'environnement.

Le travail de l'inventeur a permis de mettre en évidence qu'un concentré spécifique présentait l'ensemble des propriétés avantageuses décrites ci-dessus.

Les demandes EP1964546A1, JP2011026278A, JP2009167158A, JP2009167157A, JP2010018560A et JP2011026276A divulguent des produits cosmétiques comprenant un biosurfactant, le biosurfactant pouvant notamment être un lipide de mannosylérythritol (MEL).

L'article de TOKUMA FUKUOKA et al. "Application of yeast glycolipid biosurfactant, mannosylerythritol lipid as agrospreaders", Journal of oleo science, vol. 64, no. 6, 2015, pp. 689-695, est une étude de la mouillabilité de solutions de MEL pour des surfaces plastiques hydrophobes et des feuilles de plante.

L'article de KITAMOTO D. et al.,"Microbial conversion of n-alkanes into glycolipid biosurfactants, mannosylerythritol lipids, by Pseudozyma (Candida Antartica)", Biotechnology Letters, vol. 23, no. 20, 2001, pp. 1709-1714, décrit deux procédés de préparation de MELs grâce à la conversion de n-alcanes par *Pseudozyma antartica.*

L'article de JOSEPH IRUDAYARAJ ARUTCHELVI et al., "Mannosylerythritol lipids: a review", Journal of industrial microbiology & biotechnology, vol. 35, no. 12, 2008, pp. 1559-1570, est une revue sur les MELs, notamment concernant leurs conditions de production et leurs applications.

La demande US2013/142855A1, l'article de MNIF INES et al., "Glycolipid biosurfactants: Potential related biomedical and biotechnological applications", Carbohydrate Research, vol. 416, 2015, pp. 59-69, et l'article de N. LOURITH et al., "Natural surfactants used in cosmetics: glycolipids", International Journal of Cosmestic Science, vol. 31, no. 4, 2009, pp. 255-261 divulguent des glycolipides, dont font partie les MELs.

Aucun de ces documents ne divulgue ni ne suggère l'objet de la présente invention. La demande divulgue donc un concentré comprenant :

- au moins un lipide de mannosylérythritol, et

au moins un ester d'acide gras et de polyéthylène glycol de HLB supérieur ou égal à 12.

Selon l'invention, un « lipide de mannosylérythritol » (également appelé « MEL ») est un tensioactif appartenant à la classe des glycolipides. Plus particulièrement, un MEL est une molécule amphiphile dont la partie hydrophile est formée d'un résidu mannosylérythritol, et dont la partie hydrophobe est formée par au moins un acide gras.
Plus particulièrement, par « MEL », on désigne une molécule présentant la formule générale (I) suivante :

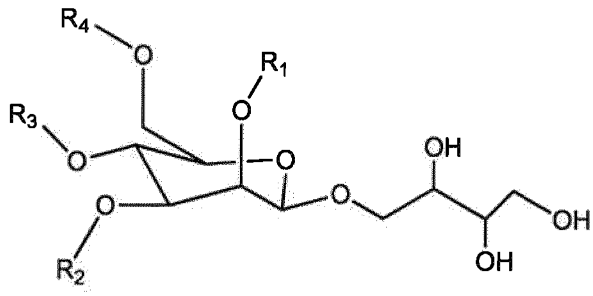

(I)

dans laquelle :

- $R_1$ et $R_2$, identiques ou différents, représentent un acide gras insaturé ou saturé, et
- $R_3$ et $R_4$, identiques ou différents, représentent un groupement acétyl ou un atome d'hydrogène.

De préférence, dans la présente invention, par « MEL », on désigne une molécule de formule (II) suivante :

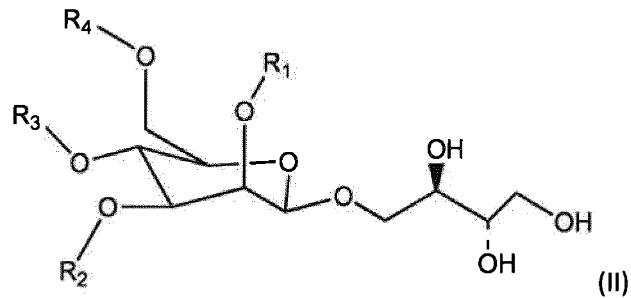

(II)

dans laquelle :

- $R_1$ et $R_2$, identiques ou différents, représentent un acide gras insaturé ou saturé, et

- $R_3$ et $R_4$, identiques ou différents, représentent un groupement acétyle ou un atome d'hydrogène.

Les formules (I) et (II) ci-avant peuvent représenter plusieurs molécules, chaque molécule étant donc un MEL. Par « MELs », on désigne au moins deux molécules de formules (I), et plus particulièrement de formule (II), différentes.
Les MELs sont généralement classés en quatre classes de molécules, notées de A à D, selon leur degré d'acétylation en $R_3$ et $R_4$. La classe des MELs-A comporte des molécules de formules (I) ou (II) présentant deux groupes acétyles en $R_3$ et $R_4$. La classe des MELs-B et la classe des MELs-C comportent des molécules de formules (I) ou (II) présentant un seul groupe acétyle en $R_4$ et $R_3$ respectivement. Enfin, la classe des MELs-D comporte des molécules de formules (I) ou (II) ne présentant pas de groupe acétyle ($R_3$= $R_4$=H).
Outre de par leur degré d'acétylation, les MELs peuvent varier dans leur structure, de par la nature des acides gras qui composent leur partie hydrophobe. Cette variation est généralement fonction du procédé mis en œuvre pour l'obtention des MELs.
Les MELs sont généralement obtenus par des procédés mettant en œuvre la culture de champignons, et plus particulièrement de levures.
Avantageusement, les MELs visés par la présente demande sont obtenus par un procédé de fermentation, comprenant les étapes suivantes :

- la culture d'une souche de champignon et plus particulièrement d'une souche de levure en présence d'une source

de carbone pour obtenir des MELs; et
- la récupération des MELs ainsi obtenus.

Les souches à partir desquelles il est possible d'obtenir des MELs sont bien connues de l'homme du métier. A titre d'exemple, il est connu d'utiliser des souches de champignons du genre *Pseudozyma* ou du genre *Ustilago*, pour l'obtention de MELs.

Avantageusement, les souches utilisées dans le procédé de fermentation décrit ci-avant, permettant l'obtention de MELs, sont des souches de champignons appartenant au genre *Pseudozyma.* De préférence, la souche est *Pzeudozyma antartica* ou *Pzeudozyma aphidis.*

De telles souches sont usuellement cultivées en réacteur dans un milieu comportant du glucose, de l'eau et/ou des sels (tel que le sulfate de magnésium, le phosphate de monopotassium et/ou le nitrate d'ammonium).

Avantageusement, les différents composants du milieu (glucose et souches en particulier) sont stérilisés séparément avant introduction dans le réacteur.

La température du milieu est de préférence comprise entre 20 et 35°C, plus préférentiellement entre 25 et 30°C.

Dans la présente demande, toutes les gammes de valeurs s'entendent bornes incluses.

Avantageusement, la source de carbone permettant la production de MELs par la souche est une huile, telle qu'une huile végétale. De préférence, la source de carbone est une huile de soja ou encore plus préférentiellement une huile de colza. Ces huiles sont particulièrement riches en acides gras comportant une chaine carbonée à 18 atomes de carbone, tels qu'en acide oléique, linoléique et/ou linolénique, ainsi que, dans une moindre mesure, en acides gras comportant une chaine carbonée à 16 atomes de carbone, tel qu'en acide palmitique.

La récupération des MELs subséquente à l'étape de culture peut comporter une étape de séparation des MELs des autres composants du milieu. Cette étape peut se faire par des méthodes de séparation classiques connues de l'homme du métier.

Avantageusement, la récupération des MELs peut comprendre une ou plusieurs des méthodes de séparation suivantes :

- la décantation ;
- l'évaporation de l'eau ou le séchage ;
- la filtration; et/ou
- la centrifugation.

Le concentré selon l'invention comporte également au moins un ester d'acide gras et de polyéthylène glycol de HLB (« Hydrophilic-Lipophilic Balance ») supérieur ou égal à 12.

Dans le cadre de la présente demande, par « polyéthylène glycol », également appelé « PEG », on entend un polymère d'oxydes d'éthylène ayant une masse molaire inférieure à 20000 $g.mol^{-1}$.

Selon l'invention, un ester d'acide gras et de polyéthylène glycol (« polyethyleneglycol fatty acid ester ») est un tensioactif non-ionique.

Le HLB permet de définir, et en particulier de chiffrer, l'équilibre entre la partie hydrophile et la partie lipophile d'une molécule de tensioactif, cet équilibre étant lié à la solubilité du tensioactif dans l'eau. La valeur de HLB peut varier de 0 à 20. Plus la valeur de HLB est élevée, plus la solubilité du tensioactif dans l'eau est grande.

Dans le cadre de la présente demande, le calcul du HLB se fait par la méthode de Griffin :

$$\text{HLB} = 20 \times \frac{\text{Masse moléculaire partie hydrophile}}{\text{Masse moléculaire de la molécule}}$$

Préférentiellement, l'ester d'acide gras et de polyéthylène glycol compris dans le concentré selon l'invention a un HLB compris entre 12 et 20, plus préférentiellement entre 12 et 16 et encore plus préférentiellement compris entre 12 et 14.

Avantageusement, le polyéthylène glycol compris dans l'ester d'acide gras et de polyéthylène glycol a une masse molaire comprise entre 200 et 4000 $g.mol^{-1}$, de préférence comprise entre 300 et 1400 $g.mol^{-1}$, plus préférentiellement comprise entre 400 et 800 $g.mol^{-1}$

L'acide gras compris dans l'ester d'acide gras et de polyéthylène glycol a avantageusement une chaine carbonée comprenant entre 8 et 24 atomes de carbones, de préférence, entre 16 et 20 atomes de carbones.

Un ester d'acide gras et de polyéthylène glycol particulièrement préféré selon l'invention est le mono-oléate de polyéthylèneglycol-600, tel que celui commercialisé par OLEON NV sous la marque RADIA® 7404.

Dans toute la présente demande, lorsqu'un nombre est indiqué derrière le terme « polyéthylène glycol- » ou « PEG- », ce nombre correspond à la masse molaire dudit polyéthylène glycol.

Plus particulièrement, l'invention concerne un concentré comprenant :

- au moins un lipide de mannosylérythritol, et
- au moins un ester d'acide gras et de polyéthylène glycol de HLB supérieur ou égal à 12,

dans lequel le ratio MEL : ester d'acide gras et de polyéthylène glycol est compris entre 0,01 et 5.
Le concentré selon l'invention possède une propriété tensioactive, et plus particulièrement lorsqu'il est mis en présence d'un liquide hydrophobe et d'eau, le concentré selon l'invention permet la formation d'une émulsion.
En effet, le concentré selon l'invention présente d'excellentes propriétés tensioactive et/ou émulsifiante, puisque lorsqu'il est ajouté à un liquide hydrophobe et à de l'eau, le concentré selon l'invention permet la formation d'une émulsion :

- uniforme, et
- de façon spontanée.

Par « de façon spontanée », on entend que seule une faible agitation est nécessaire pour obtenir une émulsion uniforme. A titre d'exemple de faible agitation, le contenant dans lequel le concentré selon l'invention, l'eau et le liquide hydrophobe sont ajoutés peut être retourné manuellement de sorte à pivoter d'un angle de 180°C, puis ramené à sa position initiale, conformément à ce qui est indiqué dans l'étape (i) de la norme CIPAC MT 36.3 (« CIPAC method 2000. Prepared by the German Formulation Panel (DAPF). Chairman : G Menschel. »*).*
Dans le cadre de la présente demande et sauf stipulation contraire, toute référence faite à une norme est une référence à la norme en vigueur à la date de dépôt.
Les propriétés tensioactive et plus particulièrement émulsifiante du concentré selon l'invention sont plus amplement décrites dans l'Exemple 2.
En outre, lorsqu'une émulsion est préparée à partir du concentré selon l'invention, celle-ci est stable dans le temps.
Par ailleurs, il a été mis en évidence par les inventeurs que, de façon surprenante, une émulsion comprenant le concentré selon l'invention a un pouvoir mouillant élevé, et possède une couleur blanche particulièrement favorable à son utilisation dans divers domaines, comme par exemple en cosmétique. Ces caractéristiques sont plus amplement décrites ci-après.
En outre, le concentré selon l'invention est stable. Par « stable », on entend que le concentré ne montre pas ou montre peu de séparation de phases après 1 jour, de préférence après 1 mois, plus préférentiellement après 2 mois de stockage.
Enfin, le concentré selon l'invention a une bonne propriété épaississante. Par « propriété épaississante », on entend qu'un concentré selon l'invention augmente la viscosité de l'eau. En d'autres termes, une composition comprenant un concentré selon l'invention et de l'eau aura une viscosité supérieure à celle de l'eau seule, avantageusement d'au moins 200 mPa.s, de préférence d'au moins 500 mPa.s, plus préférentiellement d'au moins 800 mPa.s.
Le concentré selon l'invention peut donc être utilisé en tant qu'agent épaississant.
La stabilité et la propriété épaississante du concentré selon l'invention sont plus amplement décrites dans l'Exemple 9.
Avantageusement le concentré selon l'invention comprend au moins un alcool ayant un nombre d'atomes de carbone compris entre 1 et 16.
Par « alcool », on vise plus particulièrement un alcool linéaire ou ramifié. Par « linéaire ou ramifié », on exclut spécifiquement les alcools cycliques. Plus particulièrement encore, l'alcool est un alcool constitué d'une chaîne hydrocarbonée, linéaire ou ramifiée, substituée par un ou plusieurs groupement(s) hydroxyle(s) (OH). Par « chaîne hydrocarbonée », on entend une chaîne constituée uniquement d'atomes de carbone et d'hydrogène, la chaîne hydrocarbonée comportant alors entre 1 et 16 atomes de carbone. En d'autres termes, l'alcool ne comporte pas d'hétérosubstituant autre que le ou les groupement(s) hydroxyle(s).
Le ou les alcool(s) compris dans le concentré selon l'invention permettent encore d'améliorer la stabilité et le pouvoir mouillant des émulsions formées à partir de ce concentré.
De préférence, la quantité totale d'alcool(s) dans le concentré selon l'invention est comprise entre 5 et 80% en poids, par rapport au poids total du concentré.
Par quantité totale d'alcool(s) présente dans le concentré, on entend la quantité de molécule(s) d'alcool(s) présente dans ledit le concentré, les molécules d'alcool étant telles que définies ci-avant.
De préférence, la quantité totale d'alcool(s) est comprise entre 10 et 75% en poids, plus préférentiellement entre 15 et 50% en poids, par rapport au poids total du concentré.
Avantageusement, l'alcool est saturé.
Le ou les alcool(s) sont donc linéaire(s) ou ramifié(s). Comme alcool linéaire, on peut citer l'heptanol (ou l'heptan-1-ol), l'octanol (également nommé 15 octan-1-ol ou alcool caprylique), l'alcool laurique ou le nonanol (ou nonan-1-ol ou alcool pélargonique). Comme alcool ramifié, on peut citer l'octan-2-ol, le 2-éthyl-hexanol, le 7-méthyl-octan-1-ol ou le 6-méthyl-pentan-1-ol.

Avantageusement le ou les alcool(s) compris dans le concentré selon l'invention ont une chaîne hydrocarbonée comportant un nombre d'atomes de carbone compris entre 4 et 14, préférentiellement entre 6 et 12.

De préférence, le ou les alcool(s) sont choisi(s) parmi les alcools ayant une chaîne hydrocarbonée comportant 8 à 10 atomes de carbone ou leurs mélanges. En particulier, il s'agit de l'octanol, l'octan-2-ol, le 2- éthyl-hexanol et/ou l'alcool laurique, de préférence l'octan-2-ol.

Avantageusement, l'alcool peut être obtenu à partir de ressources renouvelables, telles qu'à partir de graisses animales ou d'huiles végétales.

Le ratio MEL : ester d'acide gras et de polyéthylène glycol dans le concentré selon l'invention est compris entre 0,01 et 5.

De préférence, le ratio MEL : ester d'acide gras et de polyéthylène glycol est compris entre 0,1 et 2, plus préférentiellement entre 0,5 et 2.

Avantageusement, dans le concentré, la quantité totale de MEL(s) est comprise entre 1 et 90% en poids, par rapport au poids total du concentré.

Par quantité totale de MEL(s) présente dans le concentré, on entend la quantité de molécule(s) de MEL(s) de formules (I) ou (II) présente dans ledit concentré.

De préférence, dans le concentré selon l'invention, la quantité totale de MEL(s) est comprise entre 5 et 75% en poids, plus préférentiellement entre 10 et 50% en poids, encore plus préférentiellement entre 15 et 45% en poids, par rapport au poids total du concentré.

Avantageusement, la quantité totale d'ester(s) d'acide gras et de polyéthylène glycol dans le concentré selon l'invention est comprise entre 1 et 90% en poids, par rapport au poids total du concentré.

Par quantité totale d'ester(s) d'acide gras et de polyéthylène glycol présente dans le concentré, on entend la quantité de molécule(s) d'ester(s) d'acide gras et de polyéthylène glycol présente dans ledit concentré.

De préférence, la quantité totale d'ester(s) d'acide gras et de polyéthylène glycol est comprise entre 5 et 75% en poids, plus préférentiellement entre 10 et 65% en poids, encore plus préférentiellement entre 20 et 55% en poids, par rapport au poids total du concentré.

Avantageusement, le concentré selon l'invention comprend au moins deux MELs choisis parmi le groupe constitué par MEL-A, MEL-B, MEL-C et MEL-D.

De préférence, le concentré selon l'invention comprend des MEL(s)-A, MEL(s)-B, MEL(s)-C et optionnellement des MEL(s)-D, plus préférentiellement des MEL(s)-A, MEL(s)-B, MEL(s)-C et des MEL(s)-D.

Avantageusement, le concentré selon l'invention comprend des MELs-A et MELs-B à une teneur comprise entre 50 et 90% en poids, de préférence comprise entre 60 et 85% en poids, les pourcentages en poids étant indiqués par rapport au poids de la quantité totale de MELs.

Le concentré selon l'invention peut également comprendre des MELs-C à une teneur supérieure ou égale à 5% en poids, de préférence supérieure ou égale à 10% en poids, les pourcentages en poids étant indiqués par rapport au poids de la quantité totale de MELs.

Plus particulièrement, le concentré selon l'invention peut comprendre des MELs-A et MELs-B à une teneur comprise entre 60% et 75% en poids, et des MELs-C à une teneur supérieure ou égale à 20% en poids, les pourcentages en poids étant indiqués par rapport au poids de la quantité totale de MELs.

Le concentré selon l'invention peut comprendre en outre au moins un acide gras libre et/ou au moins un triglycéride.

Par « acide gras libre », on entend toute molécule d'acide gras qui n'est pas liée à une autre molécule. Par «acide gras», on entend toute molécule d'acide gras liée à une autre molécule, par exemple lorsque cette molécule d'acide gras est présente dans un triglycéride ou dans un MEL.

Le au moins un acide gras libre et/ou au moins un triglycéride peuvent avoir été introduits concomitamment avec le au moins un MEL.

En effet, en fonction du procédé d'obtention des MELs, tel que le procédé de fermentation décrit ci-avant, et en particulier en fonction de la ou des méthode(s) de séparation mise(s) en oeuvre dans l'étape de récupération, ceux-ci peuvent comporter un ou plusieurs acide(s) gras libre(s) et/ou triglycéride(s).

Par exemple, la quantité d'acide(s) gras libre(s) et/ou de triglycéride(s) présente dans le concentré selon l'invention peut être comprise entre 0,01 et 50% en poids, de préférence entre 0,1 et 40% en poids, par rapport au poids total du concentré.

Plus particulièrement, le concentré comporte au moins un acide gras libre et au moins un triglycéride. Dans ce cas, la quantité d'acide(s) gras libre(s) et de triglycéride(s) présente dans le concentré selon l'invention peut être comprise entre 0,01 et 50% en poids, de préférence entre 0,1 et 40% en poids, plus préférentiellement entre 1 et 35% en poids, par rapport au poids total du concentré.

Avantageusement, le ou les acide(s) gras libre(s) comporte(nt) une chaine carbonée comportant entre 14 et

24 atomes de carbone, de préférence 16 ou 18 atomes de carbone.

Avantageusement, le ou les triglycéride(s) comportent des acides gras comportant une chaine carbonée comportant entre 14 et 24 atomes de carbones, de préférence 16 ou 18 atomes de carbone.

Plus particulièrement, dans la présente demande, et en particulier dans les exemples, lorsque les MELs, à l'issue de l'étape de récupération, comportent au moins un acide gras libre, au moins un triglycéride, de l'eau et/ou des souches, ce mélange est appelé « mélange de MELs ».

Dans ce cas, le ou les acide(s) gras libre(s) et/ou triglycéride(s) peut/peuvent être issu(s) de l'huile résiduelle présente avec le ou les MEL(s) à la fin du procédé de fermentation décrit ci-avant, ladite huile résiduelle étant l'huile mis en oeuvre en tant que source de carbone dans le procédé de fermentation qui n'a pas été utilisée par les souches. De plus, le ou les acide(s) gras libre(s) peut/peuvent être issu(s) du métabolisme, par les souches, des triglycérides compris dans l'huile mise en oeuvre en tant que source de carbone dans ledit procédé.

En outre, selon le procédé d'obtention des MELs, tel que le procédé de fermentation décrit ci-avant, et en particulier selon la ou les méthode(s) de séparation mise(s) en oeuvre dans l'étape de récupération, les MELs peuvent également comporter de l'eau et des souches de champignons, plus particulièrement des souches de levures.

Selon un mode de réalisation préféré du concentré selon l'invention, celui-ci comporte un mélange de MELs présentant les caractéristiques suivantes :

- une teneur en MELs supérieure ou égale à 40% en poids, de préférence supérieure ou égale à 50% en poids, plus préférentiellement supérieure ou égale à 55% en poids ;
- une teneur en autres composants inférieure ou égale à 60% en poids, de préférence inférieure ou égale à 50% en poids, plus préférentiellement inférieure ou égale à 45% en poids (dont acides gras libres, triglycérides, eau et/ou souches),

les pourcentages en poids étant donnés par rapport au poids total du mélange de MELs.

Avantageusement, dans ce mode de réalisation préféré, la teneur en eau et/ou souches est inférieure à 3% en poids, par rapport au poids total du mélange de MELs.

Ce mélange de MELs peut être notamment obtenu selon le procédé de fermentation décrit ci-avant.

Un exemple de mélange de MELs et son procédé d'obtention est également décrit dans la publication suivante :

- "Downstream processing of mannosylerythritol lipids produced by Pseudozyma aphidis" ; Rau et al.; European Journal of Lipids Science and Technology 107 (2005) 373-380.

De préférence, un mélange de MELs comporte des MELs de différentes classes, en général au moins des MELs-A, B et C. Préférentiellement, ce mélange de MELs comporte des MELs-A, B, C et D.

En outre, un mélange de MELs comporte avantageusement des MELs-A et MELs-B à une teneur comprise entre 50 et 90% en poids, de préférence comprise entre 60 et 85% en poids, les pourcentages en poids étant indiqués par rapport au poids de la quantité totale de MELs.

De plus, un mélange de MELs comporte avantageusement des MELs-C à une teneur supérieure ou égale à 5% en poids, de préférence supérieure ou égale à 10% en poids, les pourcentages en poids étant indiqués par rapport au poids de la quantité totale de MELs.

Plus particulièrement, un mélange de MELs peut comporter des MELs-A et des MELs-B à une teneur comprise entre 60% et 75% en poids, et des MELs-C à une teneur supérieure ou égale à 20% en poids, les pourcentages en poids étant indiqués par rapport au poids de la quantité totale de MELs.

De tels mélanges de MELs sont par exemple obtenus à l'aide d'un procédé de fermentation tel que ceux décrits ci-avant.

Il est également possible d'obtenir un mélange de MELs présentant une teneur en MELs supérieure ou égale à 95%, de préférence supérieure ou égale à 98% en poids, par rapport au poids total du mélange de MELs. Ce mélange de MELs peut, par exemple, être obtenu à l'aide du procédé de fermentation décrit ci-avant auquel est ajouté une étape de purification, à l'issue de l'étape de récupération. Cette étape de purification peut comporter une extraction liquide/liquide et/ou un passage sur un substrat minéral. Le passage sur un substrat minéral peut être une chromatographie, telle qu'une chromatographie d'adsorption sur colonne de silice, réalisée à l'aide de solvants adaptés. De tels solvants sont connus de l'homme du métier.

Selon un mode de réalisation alternatif préféré du concentré selon l'invention, celui-ci peut donc comporter également un mélange de MELs qui présente les caractéristiques suivantes :

- une teneur en MELs supérieure ou égale à 95% en poids, de préférence supérieure ou égale à 98% en poids,

les pourcentages en poids étant donnés par rapport au poids total du mélange de MELs.

En outre, l'étape de purification, suite à l'étape de récupération des MELs, peut être effectuée de sorte à obtenir une classe de MELs ou même un MEL, à une teneur supérieure ou égale à 50%. A titre d'exemple, cette étape de purification peut comporter une extraction liquide/liquide et/ou un passage sur un substrat minéral (tel qu'une chromatographie), tels que définis ci-avant.

L'invention concerne également un procédé de préparation d'un concentré selon l'invention, comprenant une étape de mélangeage d'au moins un lipide de mannosylérythritol et d'au moins un ester d'acide gras et de polyéthylène glycol.

Les concentrés selon l'invention sont facilement préparables, par simple mélangeage des composants.

Avantageusement, le mélangeage est réalisé à température ambiante dans les conditions normales de température et de pression (CNTP).

De préférence, pendant le mélangeage, les composants sont chauffés à une température comprise entre 25 et 55°C, plus préférentiellement entre 30 et 50°C, encore plus préférentiellement entre 35 et 45°C.

Le chauffage des composants peut permettre une meilleure homogénéisation du concentré selon l'invention.

Optionnellement, préalablement au mélangeage, le procédé de préparation d'un concentré selon l'invention comprend l'obtention d'au moins un MEL, tel que cela est décrit ci-avant.

Avantageusement, le(s) MEL(s) est/sont tel(s) que décrit(s) ci-avant et peuvent être obtenus par le procédé de fermentation de MEL(s) décrit ci-avant, optionnellement suivi d'une étape de purification.

Avantageusement, le(s) ester(s) d'acide gras et de polyéthylène glycol utilisés dans le procédé présent(ent) les caractéristiques telles que décrites ci-avant.

L'étape de mélangeage du procédé selon l'invention peut inclure un alcool ayant un nombre d'atomes de carbone compris entre 1 et 16.

Avantageusement, le(s) alcool(s) ayant un nombre d'atomes de carbone compris entre 1 et 16 présente(nt) les caractéristiques telles que décrites ci-avant.

L'invention concerne également une composition comprenant un concentré selon l'invention, et un liquide hydrophobe.

De préférence, le liquide hydrophobe est choisi parmi l'oléate de méthyle, l'oléate d'éthyle, et les esters méthyliques ou éthyliques d'acides gras d'huile végétale, tels que les esters méthyliques ou éthyliques d'acides gras d'huile de colza, de soja, d'olive, de tournesol, de ricin, de palme et/ou de lin, ou leurs mélanges.

De préférence, le liquide hydrophobe est choisi parmi les esters méthyliques ou éthyliques d'acides gras d'huile de colza et/ou d'olive. Plus préférentiellement, le liquide hydrophobe est choisi parmi les esters méthyliques d'acides gras.

Avantageusement, la quantité de liquide hydrophobe dans la composition selon l'invention est comprise entre 50 et 99% en poids, de préférence entre 65 et 98% en poids, plus préférentiellement encore entre 80 et 96% en poids, par rapport au poids total de la composition.

L'invention concerne également un procédé de préparation d'une composition selon l'invention, comprenant une étape de mélangeage d'un concentré selon l'invention avec un liquide hydrophobe.

Avantageusement, le mélangeage est réalisé à température ambiante dans les conditions normales de température et de pression (CNTP).

De préférence, pendant le mélangeage, les composants sont chauffés à une température comprise entre 25 et 55°C, plus préférentiellement entre 30 et 50°C, encore plus préférentiellement entre 35 et 45°C.

Le chauffage des composants peut permettre une meilleure homogénéisation de la composition selon l'invention.

Avantageusement, le concentré et le liquide hydrophobe présentent les caractéristiques préférées de ces composants telles qu'elles sont décrites ci-avant.

L'invention concerne également une composition phytosanitaire comprenant un concentré selon l'invention, ou une composition selon l'invention, et un principe actif pesticide.

Avantageusement, le principe actif pesticide est choisi parmi les principes actifs herbicides, fongicides, insecticides, acaricides, régulateurs de croissance, insectifuges, de bio contrôle et/ou stimulateurs de défense des plantes.

De préférence, le principe actif pesticide est un principe actif herbicide, un principe actif fongicide, un principe actif insecticide et/ou un principe actif stimulateur de défense des plantes.

Avantageusement, la composition phytosanitaire selon l'invention comprend:

- un ou plusieurs principes actifs fongicides tel qu'un carboxamide, une strobilurine, un azole (triazole, imidazole), un composé hétérocyclique (pyridine, pyrimidine, pipérazine, morpholine), un carbamate, une huile essentielle (cinnamaldéhyde, thymol, huile de thé), un micro-organisme (champignons tel que *Gliocladium catenulatum),* levures, bactéries telle que *Bacillus subtilis)* autre que celui pouvant être compris dans le mélange de MELs décrit ci-avant, un polysaccharide (chitosan) et/ou,
- un ou plusieurs principes actifs herbicides tel qu'un inhibiteur de biosynthèse lipidique, un inhibiteur de l'acétolactase synthase (également appelé « inhibiteur d'ALS »), un inhibiteur de photosynthèse, un acétamide, un dérivé d'acides

aminés tel qu'un dérivé organophosphoré d'acide aminé (glufosinate ou glyphosate) ou leurs sels (sels d'ammonium du glufosinate, sels de mono ou di ammonium, de potassium, d'isopropylamine du glyphosate), un aryloxyphenoxypoprionate, le bipyridyl, le cyclohexanedione, une dinitroaniline, le diphenyl éther, l'hydroxybenzonitrile, l'imidazolinone , un acide phénoxy acétique, la pyrazine, la pirydine, une sulfonylurée, une triazine, une urée, un carbamate, un acide gras d'origine naturelle ayant une activité herbicide (acide caprylique, acide pélargonique) ou ses dérivés (sels, savons) et/ou,

- un ou plusieurs principes actifs insecticides tel qu'un organo(thio)phosphate, un carbamate, un pyréthrinoïde, un régulateur de croissance des insectes, un agoniste/antagoniste des récepteurs nicotiniques, un antagoniste du GABA, une lactone macrocyclique, le géraniol, l'eugénol, le thymol, l'huile de neem et/ou,
- un ou plusieurs principes actifs stimulateurs de défense des plantes, tel que *Bacillus subtilis*, un dérivé de l'acide jasmonique, un extrait d'algue.

La composition phytosanitaire selon l'invention peut être conservée longtemps avant utilisation, sans que sa teneur en principe actif pesticide ne diminue. En effet, la teneur en principe actif dans la composition phytosanitaire selon l'invention diminue très peu au cours du temps, et ce même après plusieurs mois de stockage. Cet effet est plus amplement décrit dans l'Exemple 6.

Avantageusement, le principe actif pesticide est d'origine naturelle. De tels principes actifs pesticides sont généralement appelés principes actifs bio-pesticides ou principes actifs de bio contrôle.

En outre, on notera que le principe actif compris dans la composition phytosanitaire selon l'invention peut avoir à la fois plusieurs des propriétés suivantes: herbicide, fongicide, insecticide, acaricide, régulation de croissance, insectifuge et/ou stimulateur de défense des plantes.

Avantageusement, la quantité de principe actif pesticide est comprise entre 0,1 et 30% en poids, de préférence entre 1 et 20% en poids, plus préférentiellement entre 5 et 15% en poids par rapport au poids total de la composition phytosanitaire.

L'invention concerne également une composition cosmétique comprenant un concentré selon l'invention, ou une composition selon l'invention, et un principe actif cosmétique.

Avantageusement, la composition cosmétique selon l'invention comprend un ou plusieurs principe(s) actif(s) cosmétique(s) choisi(s) parmi :

- un agent hydratant tel que l'huile de jojoba, l'huile d'amande douce, la paraffine, l'huile de germe de blé, le collagène, la pectine, le chitosan, un glycosaminoglycane et/ou,
- un filtre UV organique tel que le PABA, le PARA, un salicylate, un cinnamate, un anthranilate, le benzophenone-3, le butyl méthoxydibenzoyléthane, l'éthylhexyl triazone, le dometrizol trisiloxane, le diéthylhexyl butamido triazone, le 4-méthylbenzylidène camphor, le bemotrizinol, le diéthylamino hydroxybenzoyl hexyl benzoate, le phényl salicylate, le méthylène bis-benzotriazolyl tetramethylbutylphenol, le benzophenone-1, benzophenone-2, benzophenone-8, le bis-éthylhexyloxyphenol methoxy-phenyl triazine, ou un filtre UV minéral et/ou,
- un agent anti-âge tel qu'un rétinoïde, un $\alpha$- ou $\beta$-hydroxy acide, une vitamine hydrosoluble, le palmitate d'ascorbyle, un céramide, un pseudo céramide, un phospholipide, le cholestérol, un stérol et/ou,
- un agent anti-cellulite tel que l'isobutylmethylxanthine, la théophylline et/ou,
- agent anti-acné tel que le résorcinol, l'acétate de résorcinol, le peroxyde de benzoyl, l'acide salicylique, l'acide azélaïque et/ou,
- un agent raffermissant tel qu'un extrait de plantes (extrait de graines de lin), l'eau de rose et/ou,
- une vitamine telle que la vitamine A, ses dérivés, la vitamine B2, l'acide pantothénique, la vitamine D, la vitamine E.

Avantageusement la quantité de principe actif cosmétique est comprise entre 0,1 et 30% en poids, de préférence entre 0,5 et 20% en poids, plus préférentiellement entre 1 et 15% en poids par rapport au poids total de la composition cosmétique.

L'invention concerne également un procédé de préparation d'une composition phytosanitaire ou d'une composition cosmétique selon l'invention, comprenant une étape de mélangeage d'un concentré selon l'invention, ou d'une composition selon l'invention, avec un principe actif.

Plus particulièrement :

Dans le cadre de la préparation d'une composition phytosanitaire, le procédé selon l'invention comprend une étape de mélangeage d'un concentré selon l'invention, ou d'une composition selon l'invention, avec un principe actif pesticide ;

Dans le cadre de la préparation d'une composition cosmétique, le procédé selon l'invention comprend une étape de mélangeage d'un concentré selon l'invention, ou d'une composition selon l'invention, avec un principe actif cosmétique.

Avantageusement, les composants utilisés dans ces procédés de préparation présentent les caractéristiques préférées de ces composants telles que décrites ci-avant.

L'invention concerne en outre une émulsion comprenant une composition l'invention, une composition phytosanitaire selon l'invention ou une composition cosmétique selon l'invention, et de l'eau.

Lorsqu'une émulsion est préparée à partir d'une composition selon l'invention, d'une composition phytosanitaire selon l'invention, ou d'une composition cosmétique selon l'invention, lesquelles comprennent un concentré selon l'invention, celle-ci est stable dans le temps. Par « stable », on entend que l'émulsion ne montre pas ou montre peu de séparation de phases après 30 secondes, de préférence après 30 minutes, plus préférentiellement après 2 heures de stockage, encore plus préférentiellement après 24 heures de stockage. Avantageusement, la stabilité d'une émulsion préparée à partir du concentré selon l'invention est caractérisée conformément à l'étape (ii) de la norme CIPAC MT 36.3.

La stabilité d'émulsions préparées à partir de compositions selon l'invention comprenant un concentré selon l'invention est plus amplement décrite dans l'Exemple 3.

De plus, il a été mis en évidence par les inventeurs que, de façon surprenante, une émulsion préparée à partir d'une composition comprenant le concentré selon l'invention a un pouvoir mouillant élevé. Ainsi, lorsqu'une émulsion préparée à partir d'une composition comprenant le concentré selon l'invention est appliquée sur une surface solide (telle qu'une surface plane hydrophobe), le mouillage de cette surface solide par cette émulsion est élevé (voir Exemple 4).

Dans le cadre de la présente demande :

- par « mouillage », on entend l'étalement d'un liquide sur un solide ;
- par « tension superficielle » d'un liquide, on entend la force exercée à l'interface entre ce liquide et un solide. Les termes « tension superficielle » et « tension de surface » sont synonymes dans la présente demande ;
- par angle de contact d'une goutte de liquide 1 déposée sur une surface solide plane 2, on entend l'angle $\theta$ formé par la tangente à la goutte de liquide 1 au point de contact avec la surface solide plane 2, tel que représenté à la Figure 1.

A titre d'exemple, lorsqu'un liquide, tel qu'une goutte de solution ou d'émulsion, et un solide, tel qu'une paroi ou une feuille végétale, sont mis en contact, la capacité du liquide à mouiller le solide, c'est-à-dire à s'étaler ou se répandre sur celui-ci, dépendra directement de la force exercée à l'interface entre liquide et solide, laquelle est généralement définie comme la tension superficielle. La tension superficielle représente donc la force permettant au liquide d'adhérer au solide, ou l'empêchant de se répandre sur celui-ci. Ainsi plus la tension superficielle est importante, moins le liquide sera capable de mouiller le solide en question.

Plusieurs cas de figures peuvent donc être représentés pour illustrer la notion de mouillage. Les Figures 2a à 2c représentent plus particulièrement trois cas de figures.

Ainsi, comme illustré sur la Figure 2a, lorsqu'une goutte de liquide 1 tombe sur une surface solide plane 2, celle-ci peut réaliser un mouillage total de cette surface 2, c'est-à-dire se répandre sur toute la surface de celle-ci, en formant un film d'angle de contact $\theta$ égal à 0 avec ladite surface 2. Alternativement, la goutte 1 peut réaliser un mouillage partiel de la surface 2 (Figure 2b), c'est-à-dire ne pas se répandre totalement sur cette dernière, en formant une goutte d'angle de contact $\theta$ compris entre 0 et 90° avec ladite surface 2. Enfin, la goutte 1 peut ne pas mouiller du tout la surface 2 (Figure 2c), c'est-à-dire ne pas se répandre sur celle-ci, en formant une goutte d'angle de contact $\theta$ supérieur à 90° avec ladite surface 2.

Par ailleurs, une émulsion comprenant la composition selon l'invention présente l'avantage de pouvoir être pulvérisée sous forme de gouttelettes de diamètre avantageusement supérieur à 100$\mu$m, ce qui permet de réduire le phénomène de dérive lors de la pulvérisation.

En outre, une émulsion préparée à partir d'une composition selon l'invention comprenant le concentré selon l'invention a une couleur blanche particulièrement favorable à son utilisation dans divers domaines, comme par exemple en cosmétique. Cette caractéristique est plus particulièrement mise en évidence à la Figure 3.

L'eau est choisie en fonction de la composition à partir de laquelle l'émulsion est préparée.

Par exemple, dans le cadre d'une émulsion comprenant une composition phytosanitaire selon l'invention, l'eau est du type de celle utilisée dans la préparation d'émulsions phytosanitaires, telle qu'une eau de forage, qui peut être une eau moyenne à dure. Avantageusement, l'eau moyenne à dure présente une dureté comprise entre 300 et 600 ppm, préférentiellement entre 450 et 550 ppm. Une telle solution est généralement destinée à être pulvérisée, par exemple par un agriculteur sur des cultures.

Dans le cadre d'une émulsion comprenant une composition cosmétique selon l'invention, l'eau est une eau généralement utilisée en cosmétique, telle qu'une eau distillée ou une eau osmosée.

Avantageusement la quantité de composition, de composition phytosanitaire ou de composition cosmétique selon l'invention dans l'émulsion selon l'invention est comprise entre 0,001 et 20% en poids, de préférence entre 0,005 et 15% en poids, plus préférentiellement encore entre 0,01 et 10% en poids, par rapport au poids total de l'émulsion.

De préférence, la quantité d'eau dans l'émulsion selon l'invention est comprise entre 50 et 99,99% en poids,

de préférence entre 80 et 99,9% en poids, plus préférentiellement entre 85 et 99,9% en poids, par rapport au poids total de l'émulsion.

En particulier, la quantité d'eau peut être comprise entre 90,0 et 99,5% en poids, par rapport au poids total de l'émulsion.

Avantageusement, l'émulsion selon l'invention est une émulsion huile (ou liquide hydrophobe) dans eau.

L'invention concerne également un procédé de préparation d'une émulsion selon l'invention, comprenant une étape de mélangeage d'une composition selon l'invention, d'une composition phytosanitaire selon l'invention, ou d'une composition cosmétique selon l'invention, avec de l'eau.

L'invention concerne en outre l'utilisation d'un concentré selon l'invention, en tant qu'agent tensioactif.

En particulier, le concentré selon l'invention peut être utilisé comme émulsifiant.

Le concentré selon l'invention peut être utilisé dans toutes les applications connues des agents tensioactifs, et plus particulièrement des agents émulsifiants.

Le concentré selon l'invention peut être utilisé en tant qu'agent tensioactif dans la préparation de tout type de solutions dans lesquelles il est habituel d'utiliser des agents tensioactifs. Par « solution », dans la présente demande, on entend toute composition comprenant de l'eau telle que, par exemple, une émulsion, une dispersion ou une suspension. A titre d'exemple, le concentré selon l'invention peut être utilisé dans des solutions de nettoyage, telles que des solutions d'entretien ménager.

Avantageusement, le concentré selon l'invention est utilisé comme agent tensioactif dans une solution phytosanitaire, telle qu'une solution pesticide.

En outre, le concentré selon l'invention peut être utilisé comme agent tensioactif dans une solution cosmétique.

En outre, un concentré selon l'invention peut être utilisé dans diverses autres applications, tel que dans les pompes à incendies.

L'invention concerne par ailleurs l'utilisation d'un concentré selon l'invention en tant qu'agent épaississant.

Le concentré selon l'invention est avantageusement utilisé en tant qu'agent épaississant en cosmétique.

L'invention concerne enfin l'utilisation d'un concentré ou d'une composition selon l'invention en tant qu'adjuvant.

De préférence, l'adjuvant a une propriété mouillante.

L'invention sera mieux comprise au vu des exemples qui suivent, donnés à titre illustratif, avec référence aux Figures suivantes :

- La Figure 1, qui représente l'angle de contact $\theta$ formé par la tangente à une goutte de liquide 1 au point de contact avec une surface solide plane 2 ;
- La Figure 2, qui représente trois cas de figures illustrant la notion de mouillage, à savoir le cas d'un mouillage total d'une surface solide plane 2 par une goutte de liquide 1 (Fig. 2a), le cas d'un mouillage partiel d'une surface solide plane 2 par une goutte de liquide 1 (Fig. 2b), le cas où une goutte de liquide 1 ne mouille pas une surface solide plane 2 (Fig. 2c) ;
- La Figure 3, qui est une photographie d'une émulsion comprenant un concentré selon l'invention (portant le numéro 1 sur la photographie) et d'une émulsion comprenant un concentré comparatif (portant le numéro 3 sur la photographie) ;
- La Figure 4, qui est un diagramme représentant la diminution de l'angle de contact obtenue avec une émulsion préparée à partir d'une composition selon l'invention (comprenant un concentré selon l'invention), et avec des émulsions comprenant des compositions comparatives (comprenant des concentrés comparatifs) ;
- La Figure 5, qui est un diagramme représentant l'évolution de la teneur en principe actif pesticide au cours du temps d'une composition phytosanitaire selon l'invention, et
- La Figure 6, qui est un diagramme représentant la diminution de l'angle de contact obtenue avec une émulsion préparée à partir d'une composition selon l'invention (comprenant un concentré selon l'invention), et avec des émulsions comprenant des compositions comparatives (comprenant des concentrés comparatifs).

## Exemple 1 : Préparation de concentrés selon l'invention

### 1. Obtention de MELs

Les MELs ont été obtenus par un procédé de fermentation comprenant les étapes suivantes :

- la culture d'une souche de levure telle que *Pseudozyma aphidis* en présence d'huile végétale (colza) pour obtenir les MELs; et
- la récupération des MELs ainsi obtenus.

A l'issue de l'étape de récupération des MELs, un mélange de MELs 1A est obtenu, qui présente les caractéristiques suivantes :

- Teneur en MELs: 55% en poids
- Teneur en autres composants : 45% en poids (dont 42% en poids d'acides gras libres et de triglycérides et 3% en poids d'eau et de souche),

les pourcentages en poids étant donnés par rapport au poids total du mélange de MELs obtenu.
En particulier, le mélange de MELs 1A comporte des MELs-A à une teneur de 52% en poids, des MELs-B à une teneur de 12% en poids, des MELs-C à une teneur de 35% en poids, et des MELs-D à une teneur de 1% en poids, les pourcentages en poids étant donnés par rapport au poids de la quantité totale de MELs.

2. Ester d'acide aras et de polyéthylène glycol

Du mono-oléate de polyéthylène glycol-600 (« PEG-600-oléate ») commercialisé par OLEON NV sous la marque RADIA® 7404 a été utilisé.

3. Alcool

Du 2-octanol (Sigma-Aldrich) a été utilisé.

4. Procédé de préparation de concentrés selon l'invention

Concentré 1

Dans un flacon en verre de 60 mL, 57% en poids du mélange de MELs et 43% en poids de Radia® 7404 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

Concentré 2

Dans un flacon en verre de 60 mL, 40% en poids du mélange de MELs, 30% en poids de 2-octanol et 30% en poids de Radia® 7404 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

**Exemple 2** : **Evaluation des propriétés tensioactives et plus particulièrement émulsifiantes des concentrés selon l'invention et de concentrés comparatifs**

**1. Matériels et méthodes**

**1.1 Matériel**

Les produits qui ont été utilisés dans cet Exemple sont les suivants :

- les concentrés 1 et 2 selon l'invention préparés à l'Exemple 1
- du mono-oléate de polyéthylène glycol-600 (Radia@ 7404, OLEON NV, HLB = 13,2)
- le mélange de MELs préparé à l'Exemple 1
- des esters méthyliques d'acides gras d'huile de colza (Radia@ 7955, OLEON NV)
- de l'eau standard C (Préparée selon la norme CIPAC MT 18.1) Le matériel suivant a également été utilisé dans cet Exemple :
- des flacons en verre de 60 mL
- des éprouvettes graduées de 100 mL
- des pipettes graduées de 5 mL.

### 1.2. Méthodes

Concentrés selon l'invention

Les concentrés 1 et 2 préparés à l'Exemple 1 ont été utilisés.

Préparation des concentrés comparatifs

Concentré 3 comparatif
Le concentré 3 comprend uniquement du Radia® 7404.

Concentré 4 comparatif

Le concentré 4 comprend uniquement le mélange de MELs préparé à l'Exemple 1.

Evaluation des propriétés tensioactives des concentrés 1 à 4

Un protocole de préparation d'émulsions à partir des concentrés 1 et 2 selon l'invention et des concentrés 3 et 4 comparatifs a été réalisé, conformément à l'étape (i) de la norme CIPAC MT 36.3 (« CIPAC method 2000. Prepared by the German Formulation Panel (DAPF). Chairman : G Menschel. »).
Dans un premier temps, les concentrés 1 à 4 sont mis en contact avec un liquide hydrophobe selon le protocole suivant :
Pour se faire:

- dans un flacon, 7% en poids du concentré 1 et 93% en poids de Radia® 7955 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation,
- dans un flacon, 10% en poids du concentré 2 et 90% en poids de Radia® 7955 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation,
- dans un flacon, 3% en poids du concentré 3 et 97% en poids de Radia® 7955 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation,
- dans une éprouvette graduée, 4% en poids du concentré 4 et 96% en poids de Radia® 7955 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation.

On obtient ainsi les compositions 1 à 4.
Ces compositions 1 à 4 sont ensuite mises en contact avec de l'eau selon le protocole suivant :
Dans des éprouvettes graduées, 1% en poids des différentes compositions 1 à 4 a respectivement été ajouté à 99 % en poids d'eau standard, les % en poids étant indiqués par rapport au mélange eau-préparation. Les éprouvettes graduées ont été refermées à l'aide d'un bouchon. Les éprouvettes graduées ont ensuite été retournées une fois. Conformément à la note 4 de la norme CIPAC MT 36.3, lorsqu'une éprouvette graduée est « retournée une fois », on entend que l'éprouvette graduée est retournée manuellement de sorte à pivoter d'un angle de 180°C, puis ramenée à sa position initiale, le tout en environ deux secondes.
Après 30 secondes, il a été observé à l'œil nu si une émulsion s'est formée ou non dans chaque éprouvette graduée. Lorsqu'une émulsion s'est formée, il a été observé si celle-ci était uniforme (complète), ou au contraire incomplète. Lorsqu'une émulsion uniforme se forme, cela signifie que le concentré présente une excellente propriété tensioactive. Au contraire, lorsque l'émulsion est incomplète, cela signifie que le concentré ne présente pas une bonne propriété tensioactive.
Les résultats sont présentés dans le Tableau 1 ci-dessous.

Tableau 1 : Propriétés tensioactive et émulsifiante des concentres 1 a 4

| Concentré | Observation à 30 secondes |
|---|---|
| Concentré 1 selon l'invention | Emulsion uniforme |

(suite)

| Concentré | Observation à 30 secondes |
|---|---|
| Concentré 2 selon l'invention | Emulsion uniforme |
| Concentré 3 comparatif | Emulsion uniforme |
| Concentré 4 comparatif | Pas d'émulsion formée |

Les résultats présentés dans le Tableau 1 montrent que le concentré selon l'invention, lorsqu'il est mis en présence d'eau et d'un liquide hydrophobe, permet la formation d'émulsions uniformes, et ce de façon spontanée (après une faible agitation : un seul retournement manuel). Les concentrés selon l'invention ont donc une excellente propriété tensioactive et plus particulièrement émulsifiante.

Les émulsions 1 à 3 préparées à partir des concentrés 1 à 3 ont été utilisées dans l'Exemple 3 ci-après.

**Exemple 3 : Evaluation de la stabilité d'émulsions préparées à partir de concentrés selon l'invention et d'un concentré comparatif**

La stabilité des émulsions 1 à 3 préparées dans l'Exemple 2 a été évaluée, conformément à l'étape (ii) de la norme CIPAC MT 36.3. Lorsqu'une émulsion est stable, celle-ci apparaît sous la forme d'une seule phase. Au contraire, lorsqu'une émulsion est instable, il peut être observé à l'œil nu une séparation de la phase huileuse et de la phase aqueuse. Cette séparation de phases se traduit par la présence de crème, qui représente la phase aqueuse en cours de séparation, et d'huile libre, qui représente la phase huileuse en cours de séparation. Le niveau de séparation de la phase huileuse et de la phase aqueuse peut être quantifié par les volumes de crème et d'huile libre présents dans l'émulsion. En outre, la préparation d'une émulsion peut résulter en la formation de mousse. A l'échelle industrielle, l'inconvénient de la formation de mousse lors de la préparation d'une émulsion phytosanitaire est que l'utilisateur ne pourra verser la quantité d'eau requise pour la préparation de cette émulsion, ce qui peut résulter en une trop forte concentration en principe actif phytosanitaire, ou même à un débordement de la cuve contenant l'émulsion. La quantité de mousse peut être quantifiée par le volume de mousse présent dans l'émulsion.

Evaluation de la stabilité des émulsions 1 à 3

Suite à l'observation à 30 secondes des émulsions 1 à 3 de l'Exemple 2, les éprouvettes contenant ces émulsions ont été retournées dix fois puis déposées dans une pièce où elles sont restées pendant 24 heures à une température constante de 20+/- 2°C. A 30 minutes, à 1 heure, à 2 heures et à 24 heures, les volumes d'huile libre et/ou de crème formée en haut ou en bas des émulsions, ont été mesurés par lecture du volume correspondant sur les éprouvettes graduées. Les volumes de mousse ont également été mesurés de la même manière.

Résultats

Les résultats sont présentés dans le Tableau 2 ci-après.

Tableau 2 : Stabilité des musons 1 à 3

| | Emulsion 1 | | | Emulsion 2 | | | Emulsion 3 | | |
|---|---|---|---|---|---|---|---|---|---|
| | Mousse (mL) | Crème (mL) | Huile (mL) | Mousse (mL) | Crème (mL) | Huile (mL) | Mousse (mL) | Crème (mL) | Huile (mL) |
| 30 minutes | 1 | 0 | 0 | 1 | 0 | 0 | 3 | 0,5 | 0 |
| 1 heure | 1 | 0 | 0 | 0,5 | 0 | 0 | 2 | 1 | 0 |
| 2 heures | 0 | 1 | 0,2 | 0,5 | 1 | 0 | 1 | 1 | 0 |
| 24 heures | 0 | 0 | 1 | 0 | 1 | 0 | 0 | 2 | 0 |

Les résultats présentés dans le Tableau 2 montrent que les émulsions 1 et 2 selon l'invention présentent une meilleure stabilité que l'émulsion 3 comparative. En particulier, l'apparition de crème dans les émulsions 1 et 2 selon

l'invention est moins rapide que celle dans l'émulsion 3 comparative.

Par ailleurs, les émulsions 1 et 2 selon l'invention comportent moins de mousse que l'émulsion 3 comparative.

De plus, en comparant les émulsions 1 et 2, on note, après 24h, qu'il y a apparition d'huile en surface de l'émulsion 1 ne comprenant pas d'alcool. Ce phénomène n'est pas observable pour l'émulsion 2, qui est de composition identique à celle de l'émulsion 1 mais qui comprend également un alcool. Par conséquent, ce dernier permet de mieux stabiliser l'émulsion dans le temps.

En outre, une émulsion comprenant un concentré selon l'invention a une couleur très blanche, ce qui la rend particulièrement adaptée à une utilisation cosmétique.

A titre d'exemple, des photographies de l'émulsion 1 comprenant le concentré 1 selon l'invention (portant le numéro 1 sur la photographie) et de l'émulsion 3 comprenant le concentré 3 comparatif (portant le numéro 3 sur la photographie) sont présentées à la Figure 3. Ces photographies ont été prises 5 minutes après avoir retourné dix fois les éprouvettes contenant ces émulsions.

## Exemple 4 : Evaluation du pouvoir mouillant d'émulsions préparées à partir d'un concentré selon l'invention et de concentrés comparatifs

### 1. Matériels et Méthodes

#### 1.1. Matériel

Les produits suivants ont été utilisés :

- le mélange de MELs préparé à l'Exemple 1 (« MEL »)
- des esters méthyliques d'acides gras d'huile de colza (« EMC », Radia® 7955, OLEON NV)
- du mono-oléate de polyéthylène glycol-600 (« PEG-600-oléate », Radia® 7404, OLEON NV)
- du polysorbate 20 (Radia@ 7137, OLEON NV, HLB : 16,5)
- du polysorbate 80 (Radia@ 7157, OLEON NV, HLB : 14,9)
- de l'eau standard C

Le matériel suivant a été utilisé:

- des flacons en verre,
- une seringue de 1 mL munie d'une aiguille de diamètre 0,63 mm
- un parafilm hydrophobe (Parafilm « M », NEENAH, WI 54956)
- le goniomètre DSA10 (KRUSS)
- le logiciel « Drop shape analysis » (KRUSS)

### 2. Méthodes

#### Préparations du concentré selon l'invention

Le concentré 1 de l'Exemple 1 a été utilisé.

#### Préparation des concentrés comparatifs

Concentré 5 comparatif

Dans un flacon en verre de 60 mL, 57% en poids du mélange de MELs et 43% en poids de Polysorbate 20 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

Concentré 6 comparatif

Dans un flacon en verre de 60 mL, 57% en poids du mélange de MELs et 43% en poids de Polysorbate 80 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

Préparation d'émulsions à partir des concentrés 1, 5 et 6

Un protocole de préparation d'émulsions à partir du concentré 1 selon l'invention et des concentrés 5 et 6 comparatifs a été réalisé, conformément à l'étape (i) de la norme CIPAC MT 36.3 (« CIPAC method 2000. Prepared by the German Formulation Panel (DAPF). Chairman : G Menschel. »*).*

Pour ce faire, dans un premier temps, les concentrés 1, 5 et 6 sont mis en contact avec un liquide hydrophobe selon le protocole suivant :

Dans des flacons, 7% en poids des différents concentrés 1, 5 et 6 et 93% en poids de Radia® 7955 ont étés ajoutés, les % en poids étant indiqués par rapport au poids total de chaque composition obtenue, puis agités manuellement jusqu'à homogénéisation de la composition. Lors de l'agitation, il est possible de chauffer les compositions à 40°C pour faciliter l'homogénéisation. On obtient ainsi les compositions 1, 5 et 6.

Ces compositions 1, 5 et 6 sont ensuite mises en contact avec de l'eau selon le protocole suivant :

Dans des flacons, 1% en poids des différentes compositions 1, 5 et 6 obtenues a respectivement été ajouté à 99% en poids d'eau standard, les % en poids étant indiqués par rapport au poids total du mélange eau-composition. Les flacons ont été refermés à l'aide d'un bouchon. Les flacons ont ensuite été retournés une fois. Les émulsions 1, 5 et 6 ont été obtenues.

Mesure des angles de contact

Des mesures d'angle de contact ont été réalisées pour chacune des émulsions 1, 5 et 6 et pour la solution contrôle, à l'aide du goniomètre.

Pour cela, une goutte de chaque émulsion ou de la solution contrôle (3µL) a été formée à l'aide de la seringue. La seringue a ensuite été placée à environ 0,5 cm au-dessus du parafilm hydrophobe. Par la pesanteur, cette goutte s'est détachée de l'aiguille et est tombée sur le parafilm hydrophobe. Le suivi de la variation de l'angle de contact a été assuré dès le moment où la goutte a touché le parafilm, et ce pendant 10 minutes, grâce au logiciel d'analyse.

Les résultats ont ensuite été traités afin de comparer les variations d'angles de contact pour chacune des émulsions 1, 5 et 6, par rapport à la solution contrôle.

## 2. Résultats

Les résultats des mesures d'angle de contact pour chacune des émulsions 1, 5 et 6 et de la solution contrôle sont présentés à la Figure 4.

Les résultats montrent que la diminution de l'angle de contact obtenue avec l'émulsion 1 est notablement supérieure à celle obtenue avec chacune des émulsions 5 et 6.

## Exemple 5 : Utilisation d'un concentré selon l'invention dans la préparation d'une composition phytosanitaire - Préparation d'une émulsion contenant une huile essentielle

Le concentré 2 selon l'invention préparé à l'Exemple 1 a été utilisé dans la préparation d'une composition phytosanitaire.

Le concentré 2 présente les caractéristiques suivantes :

| Composants | % en poids* |
|---|---|
| Mélange de MELs | 40 |
| Radia® 7404 | 30 |
| 2-octanol | 30 |
| *Pourcentage en pois par rapport au pois total du concentre. | |

La composition phytosanitaire a ensuite été préparée, par simple mélangeage du concentré 2 selon l'invention avec les 3 autres composants décrits dans le tableau ci-après. La composition phytosanitaire selon l'invention obtenue présente les caractéristiques suivantes :

| Composants | % en poids* |
|---|---|
| Concentré 2 selon l'invention | 10% |

(suite)

| Composants | % en poids* |
|---|---|
| Radia® 7955 | 79,95% |
| Cinnamaldéhyde (Herbarom laboratoire) | 10% |
| Eugénol (Sigma-Aldrich) | 0,05% |
| *Pourcentage en poids par rapport au poids total de la composition phytosanitaire. | |

Une émulsion a ensuite été préparée à partir de la composition phytosanitaire selon l'invention obtenue, comme suit :
Dans un récipient refermable, 50% en poids d'eau, 1% en poids de la composition phytosanitaire selon l'invention, puis 49% en poids d'eau ont successivement été ajoutés, les pourcentages en poids étant indiqués par rapport au poids total du mélange eau-composition. Le récipient a ensuite été refermé puis retourné pour obtenir l'émulsion. Si nécessaire, le pH et la salinité de l'eau auront été ajusté au préalable.
L'émulsion selon l'invention obtenue présente les caractéristiques suivantes :

| Composants | % en poids* |
|---|---|
| Composition phytosanitaire selon l'invention | 1% |
| Eau | 99% |
| *Pourcentage en poids par rapport au poids total de l'émulsion. | |

**Exemple 6 : Evaluation de la teneur en principe actif pesticide au cours du temps dans une composition phytosanitaire selon l'invention**

L'évolution de la teneur en cinnamaldéhyde au cours du temps de la composition phytosanitaire selon l'invention préparée dans l'Exemple 5 a été analysée par chromatographie en phase gazeuse (analyse GC).

1. Matériel

Le matériel suivant a été utilisé :

- une balance de précision à 0,00001 g
- des pipettes automatiques de 1 et 5 mL
- des pipettes Pasteur
- des fioles jaugées de 20 et 100 mL
- un chromatographe pour chromatographie en phase gazeuse (GC 6580 - Agilent Technologies)
- une seringue de 5µL

2. Méthodes

La composition phytosanitaire selon l'invention préparée dans l'Exemple 5 a été stockée pendant deux mois au cours desquels une analyse GC a été réalisée à différents temps (fabrication, 0 jours, 15 jours, 1 mois, 2 mois, 3 mois).
Le stockage a été réalisé :

- à température ambiante (environ 20°C en conditions normales de température et de pression (CNTP)), ou
- à 54°C.

Les paramètres de l'analyse GC sont les suivants :

- injecteur : split mode ; température : 350°C ; split ratio : 10 :1
- phase mobile : Hélium
- débit gaz vecteur : 2 mL/min

- temps d'analyse : 36 minutes
- volume d'injection : 2µL
- détecteur : FID ; température : 250°C ; air : 450 mL/min ; H2 : 40 mL/min ; He : 30 mL/min
- colonne : DB5 HT 15m*250µm*0,1µm

Tous les composants utilisés dans la préparation de la composition phytosanitaire selon l'invention ont été passés seuls en GC selon les paramètres GC ci-dessus. Ceci a permis de vérifier qu'aucun de ces composants n'avait un temps de rétention identique au cinnamaldéhyde ou à l'étalon interne (azulène).
Plus particulièrement :

- le temps de rétention du cinnamaldéhyde est de 6 min.
- le temps de rétention de l'azulène est de 6,7 min.
- les composants ont des temps de rétention différents de ceux du cinnamaldéhyde et de l'azulène.

La sélectivité de la méthode est conforme.
Lors d'une 1$^{ère}$ étape, l'étalonnage interne avec des solutions de cinnamaldéhyde à différentes concentrations a été réalisé.
Ensuite, la 2$^{ème}$ étape consistant en l'analyse GC a été réalisée, suivi du calcul du taux de cinnamaldéhyde dans la composition phytosanitaire, à l'aide de la courbe étalon.
Les résultats de l'analyse GC sont présentés dans le Tableau 4 ci-dessous et à la Figure 5.

Tableau 4 : Résultats de l'analyse GC de la composition phytosanitaire selon l'invention

| Temps | Teneur en cinnamaldéhyde | | Limites min 10% | Limites max 10% |
|---|---|---|---|---|
| | Température ambiante | 54 °C | | |
| Fabrication | 10 | 10 | 9 | 11 |
| 0 jours | 9,71 | 9,71 | 9 | 11 |
| 15 jours | 9,91 | 10,07 | 9 | 11 |
| 1 mois | 9,89 | 9,88 | 9 | 11 |
| 2 mois | 9,74 | 9,57 | 9 | 11 |
| 3 mois | 10,22 | 9,81 | 9 | 11 |

Les résultats montrent que la teneur en cinnamaldéhyde de la composition phytosanitaire selon l'invention diminue très faiblement au cours du temps, et ce quelles que soient les conditions de stockage.

**Exemple 7 : Utilisation d'un concentré selon l'invention dans la préparation d'une composition cosmétique - Préparation d'une émulsion**

Le concentré 1 selon l'invention préparé à l'Exemple 1 a été utilisé dans la préparation d'une composition cosmétique.
Le concentré 1 présente les caractéristiques suivantes :

| Composants | % en poids* |
|---|---|
| Mélange de MELs | 57% |
| Radia® 7404 | 43% |
| *Pourcentage en poids par rapport au poids total ou concentré. | |

La composition cosmétique a ensuite été préparée, par simple mélangeage du concentré selon l'invention avec les 2 autres composants décrits dans le tableau ci-dessous. La composition cosmétique selon l'invention obtenue présente les caractéristiques suivantes :

| Composants | % en poids* |
|---|---|
| Concentré 1 selon l'invention | 7% |
| Methyl olivate (PEL-EST® OME - Elé corporation) | 91% |
| Huile de jojoba SCB (Lucas Meyer cosmetics) | 2% |
| *Pourcentage en poids par rapport au poids total de la composition cosmétique. | |

Une émulsion a ensuite été préparée à partir de la composition cosmétique selon l'invention obtenue, comme suit :
Dans un flacon, 50% en poids d'eau, 5% en poids de la composition cosmétique selon l'invention, et 45% en poids d'eau ont successivement été ajoutés, les pourcentages en poids étant indiqués par rapport au poids total du mélange eau-composition. Le flacon a ensuite été refermé puis retourné pour obtenir l'émulsion. Si nécessaire, le pH et la salinité de l'eau auront été ajusté au préalable.

L'émulsion selon l'invention obtenue présente les caractéristiques suivantes :

| Composants | % en poids* |
|---|---|
| Composition cosmétique selon l'invention | 5% |
| Eau | 95% |
| *Pourcentage en pois par rapport au pois total de l'émulsion. | |

D'autres composants peuvent être ajoutés à cette émulsion, tels que d'autres principes actifs cosmétiques et/ou des agents de formulation, ces derniers permettant notamment de lui conférer les propriétés de texture (crème, gel) et/ou sensorielles souhaitées.

**Exemple 8 : Evaluation de la stabilité et du pouvoir mouillant d'émulsions préparées à partir d'un concentré selon l'invention et de concentrés comparatifs**

1. Matériels et Méthodes

1.1. Matériel

Les produits suivants ont été utilisés :

- Le concentré 2 selon l'invention préparé à l'Exemple 1
- du polyglycérol-3 caprylate/caprate (PG-3-C8/C10, C094, OLEON)
- du Simulsol® SL11W (SEPPIC)
- des esters méthyliques d'acides gras d'huile de colza (« EMC », Radia® 7955, OLEON NV)
- du 2-octanol (Sigma-Aldrich)
- du mono-oléate de polyéthylène glycol-600 (Radia@ 7404, OLEON)
- de l'eau standard D (préparée selon la norme CIPAC MT 18.1.4)

Le matériel suivant a été utilisé:

- des flacons en verre,
- des éprouvettes graduées,
- des pipettes graduées
- une seringue de 1 mL munie d'une aiguille de diamètre 0,63 mm
- un parafilm hydrophobe (Parafilm « M », NEENAH, WI 54956)
- le goniomètre DSA10 (KRUSS)
- le logiciel « Drop shape analysis » (KRUSS)

1.2. Méthodes

Préparation du concentré selon l'invention

Le concentré 2 de l'Exemple 1 a été utilisé.

Préparation des concentrés comparatifs

Concentré comparatif 7 :

Dans un flacon en verre de 120 mL, 40% en poids de polyglycérol-3 caprylate/caprate, 30% en poids de 2-octanol et 30% en poids de mono-oléate de polyéthylène glycol-600 ont été ajoutés, les % en poids étant indiqués par rapport au poids total du concentré obtenu, puis agités manuellement jusqu'à homogénéisation du concentré. Lors de l'agitation, le concentré est chauffé à approximativement 50°C pour faciliter l'homogénéisation.

Concentré comparatif 8 :

Dans un flacon en verre de 120 mL, 40% en poids de Simulsol® SL11W, 30% en poids de 2-octanol et 30% en poids de mono-oléate de polyéthylène glycol-600 ont été ajoutés, les % en poids étant indiqués par rapport au poids total du concentré obtenu, puis agités manuellement jusqu'à homogénéisation du concentré. Lors de l'agitation, le concentré est chauffé à approximativement 50°C pour faciliter l'homogénéisation.

Préparation d'émulsions à partir des concentrés 2, 7 et 8

Un protocole de préparation d'émulsions à partir du concentré 2 selon l'invention et des concentrés 7 et 8 comparatifs a été réalisé, conformément à l'étape (i) de la norme CIPAC MT 36.3 (« CIPAC method 2000. Prepared by the German Formulation Panel (DAPF). Chairman : G Menschel. »).

Pour ce faire, dans un premier temps, les concentrés 2, 7 et 8 sont mis en contact avec un liquide hydrophobe selon le protocole suivant :

Dans des éprouvettes, 10% en poids des différents concentrés 2, 7 et 8 et 90% en poids de Radia® 7955 ont étés ajoutés, les % en poids étant indiqués par rapport au poids total de chaque composition obtenue, puis agités manuellement jusqu'à homogénéisation de la composition. Lors de l'agitation, il est possible de chauffer les compositions à 40°C pour faciliter l'homogénéisation. On obtient ainsi les compositions 2, 7 et 8.

Il est observé que les compositions comparatives 7 et 8 sont troubles à température ambiante, ainsi qu'après chauffage à 50°C. De plus, pour ces deux compositions, un dépôt est observé au fond du flacon 24 heures après agitation. Au contraire, la composition 2 selon l'invention est homogène à température ambiante, ainsi qu'après chauffage à 50°C. Aucun dépôt n'est observé au fond du flacon 24 heures après agitation.

Ces compositions 2, 7 et 8 sont ensuite mises en contact avec de l'eau selon le protocole suivant :

Dans des éprouvettes, 1% en poids des différentes compositions 2, 7 et 8 obtenues a respectivement été ajouté à 99% en poids d'eau standard, les % en poids étant indiqués par rapport au poids total du mélange eau-composition. Les flacons ont été refermés à l'aide d'un bouchon. Les flacons ont ensuite été retournés une fois.

Après 30 secondes, il a pu être observé à l'œil nu qu'une émulsion uniforme s'est formée dans chaque éprouvette graduée. Des émulsions non-uniformes (incomplètes) se sont formées à partir des compositions comparatives 7 et 8.

Evaluation de la stabilité des émulsions 2, 7 et 8

La stabilité des émulsions 2, 7 et 8 préparées a été évaluée, conformément à l'étape (ii) de la norme CIPAC MT 36.3.

Suite à l'observation à 30 secondes des émulsions 2, 7 et 8, les éprouvettes contenant ces émulsions ont été retournées dix fois puis déposées dans une pièce où elles sont restées pendant 24 heures à une température constante de 20+/- 2°C. A 30 minutes, à 1 heure, à 2 heures et à 24 heures, les volumes d'huile libre et/ou de crème formée en haut ou en bas des émulsions, ont été mesurés par lecture du volume correspondant sur les éprouvettes graduées. Les volumes de mousse ont également été mesurés de la même manière.

Résultats

Les résultats sont présentés dans le Tableau 5 ci-après.

Tableau 5 : Stabilité des émulsions 2, 7 et 8

| | Emulsion 2 | | | Emulsion 7 | | | Emulsion 8 | | |
|---|---|---|---|---|---|---|---|---|---|
| | Mousse (mL) | Crème (mL) | Huile (mL) | Mousse (mL) | Crème (mL) | Huile (mL) | Mousse (mL) | Crème (mL) | Huile (mL) |
| 30 minutes | 1 | 0 | 0 | 3 | 1 | 0 | 2 | 0,5 | 0,5 |
| 1 heure | 1 | 0 | 0 | 1 | 1 | 0 | 1 | 1 | 1 |
| 2 heures | 0 | 1 | 0,2 | 1 | 1 | 0 | 1 | 1 | 1 |
| 24 heures | 0 | 0 | 1 | 0 | 2 | 0 | 0 | 2 | 2 |

Les résultats présentés dans le Tableau 5 montrent que l'émulsion 2 selon l'invention présentent une meilleure stabilité que les émulsions 7 et 8 comparatives. En particulier, l'apparition de crème dans l'émulsion 2 selon l'invention est moins rapide que celle dans les émulsions 7 et 8 comparatives, et il peut être observé que la crème n'est plus présente dans l'émulsion 2 à 24 heures.

2. Evaluation du pouvoir mouillant des émulsions 2, 7 et 8

Mesure des angles de contact

Des mesures d'angle de contact ont été réalisées pour chacune des émulsions 2, 7 et 8 et pour et pour une solution contrôle d'eau standard D, à l'aide du goniomètre.
Pour cela, une goutte de chaque émulsion ou de la solution contrôle (3μL) a été formée à l'aide de la seringue. La seringue a ensuite été placée à environ 0,5 cm au-dessus du parafilm hydrophobe. Par la pesanteur, cette goutte s'est détachée de l'aiguille et est tombée sur le parafilm hydrophobe. Le suivi de la variation de l'angle de contact a été assuré dès le moment où la goutte a touché le parafilm, et ce pendant 10 minutes, grâce au logiciel d'analyse.
Les résultats ont ensuite été traités afin de comparer les variations d'angles de contact pour chacune des émulsions 2, 7 et 8, par rapport à la solution contrôle.

Résultats

Les résultats des mesures d'angle de contact pour chacune des émulsions 2, 7 et 8 et de la solution contrôle sont présentés à la Figure 6.
Les résultats montrent notamment que la diminution de l'angle de contact obtenue avec l'émulsion 2 est notablement supérieure à celle obtenue avec chacune des émulsions 7 et 8.

**Exemple 9 : Evaluation de la stabilité et de la propriété épaississante de concentrés selon l'invention et de concentrés comparatifs**

1. Obtention de MELs

Un mélange de MELS 1A a été obtenu par le procédé décrit à l'Exemple 1.
Une étape de purification du mélange de MELs 1A a ensuite été réalisée par chromatographie d'adsorption sur colonne de silice, avec utilisation d'un mélange de solvants ayant un gradient de polarité croissant. Un second mélange de MELs (mélange de MELs 1B) a ainsi été obtenu, qui présente les caractéristiques suivantes :

- Teneur en MELs: au moins 98% en poids, par rapport au poids total du mélange de MELs obtenu.

En particulier, le mélange de MELs 1B comporte des MELs-A à une teneur de 52% en poids, des MELs-B à une teneur de 12% en poids, des MELs-C à une teneur de 35% en poids, et des MELs-D à une teneur de 1% en poids, les pourcentages en poids étant donnés par rapport au poids de la quantité totale de MELs.

2. Ester d'acide gras et de polyéthylène glycol

Du mono-oléate de polyéthylène glycol-600 (« PEG-600-oléate ») commercialisé par OLEON NV sous la marque RADIA® 7404 a été utilisé.

3. Préparation des concentrés selon l'invention et des concentrés comparatifs

Concentré 9 selon l'invention

Dans un flacon en verre de 60 mL, 10% en poids du mélange de MELs 1B et 90% en poids de Radia® 7404 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

Concentré 10 selon l'invention

Dans un flacon en verre de 60 mL, 50% en poids du mélange de MELs 1B et 50% en poids de Radia® 7404 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

Concentré 11 selon l'invention

Dans un flacon en verre de 60 mL, 67% en poids du mélange de MELs 1B et 33% en poids de Radia® 7404 ont été ajoutés, les % en poids étant indiqués par rapport au poids total du concentré obtenu, puis agités manuellement jusqu'à homogénéisation du concentré. Lors de l'agitation, il est possible de chauffer le concentré à 40°C pour faciliter l'homogénéisation.

Concentré 12 comparatif

Le concentré 12 ne comprend que du Radia® 7404.

Concentré 13 comparatif

Le concentré 13 ne comprend que le mélange de MELs 1B.

4. Evaluation de la stabilité des concentrés 9 à 13

La stabilité des concentrés 9 à 13 a été évaluée après 1 jour et après 2 mois de stockage.

Lorsqu'un concentré est stable, celui-ci apparaît sous la forme d'une seule phase. Au contraire, lorsqu'un concentré est instable, il peut être observé à l'œil nu une séparation de phases entre les différents composants de ce concentré.

La stabilité a été évaluée par inspection à l'œil nu.

Les résultats sont présentés dans le Tableau 6 ci-dessous.

Tableau 6 : Stabilité des concentres 9 a 13 préparés dans l'Exemple 9 après 1 jour et après 2 mois de stockage

| Concentré | Stabilité après 1 jour | Stabilité après 2 mois |
|---|---|---|
| Concentré 9 | une phase homogène | une phase légérement trouble |
| Concentré 10 | une phase homogène | une phase homogène |
| Concentré 11 | une phase homogène | une phase homogène |
| Concentré 12 (comparatif) | une phase homogène | Non évaluée |
| Concentré 13 (comparatif) | deux phases | Non évaluée |

Les résultats montrent que les concentrés 9 à 11 comprenant un concentré selon l'invention sont stables après

2 mois de stockage.

5. Evaluation de la propriété épaississante des concentrés 9 à 13

Dans des flacons en verre, 10% en poids des concentrés 9 à 11 selon l'invention et des concentrés 12 et 13 comparatifs puis 90% en poids d'eau ont été respectivement ajoutés, les % en poids étant indiqués par rapport au poids total de chaque mélange concentré/eau obtenu. L'ajout de l'eau dans les flacons comprenant les différents concentrés se fait sous agitation manuelle avec une spatule.

La viscosité dynamique des mélanges 9 à 13 obtenus a été évaluée.

La viscosité est évaluée à l'aide d'un réhomètre (TA instruments AR 2000) à 25°C, à une vitesse de 10 rpm.

La viscosité dynamique de l'eau (contrôle) est de 1 mPA.s.

Les résultats sont présentés dans le Tableau 7 ci-dessous.

Tableau 7 : Viscosités dynamique des mélanges 9 à 13 préparés dans l'Exemple 9

| Mélange | Viscosité (mPA.s) |
|---|---|
| Mélange 9 | 1140 |
| Mélange 10 | 1270 |
| Mélange 11 | 1240 |
| Mélange 12 (comparatif) | 33 |
| Mélange 13 (comparatif) | Non applicable |

Les résultats montrent que les mélanges 9 à 11 comprenant un concentré selon l'invention et de l'eau ont une vicosité dynamique nettement supérieure à celle de l'eau pure. Au contraire, les mélanges 12 et 13 comprenant des concentrés comparatifs ont une viscosité proche de celle de l'eau.

Un concentré selon l'invention possède une bonne propriété épaississante, et peut donc être utilisé en tant qu'agent épaississant.

**Exemple 10 : Evaluation de l'émulsionnabilité de compositions comparatives comprenant un tensioactif non-ionique de HLB inférieur à 12**

**1. Matériels et méthodes**

**1.1 Matériel**

Les produits qui ont été utilisés dans cet Exemple sont les suivants :

- le mélange de MELs 1A préparé à l'Exemple 1
- des esters méthyliques d'acides gras d'huile de colza (Radia® 7955, OLEON NV)
- du di-oléate de polyéthylène glycol-600 (Radia® 7444, OLEON NV, HLB : 10)
- du mono-oléate de polyéthylène glycol-200 (Radia® 7402, HLB : 7)
- du 2-octanol (Sigma-Aldrich)
- de l'eau standard D (préparée selon la norme CIPAC MT 18.1.4)

Le matériel suivant a également été utilisé dans cet Exemple :

- des flacons en verre de 60 mL
- des éprouvettes graduées de 100 mL
- des pipettes graduées de 5 mL

### 1.2. Méthodes

Préparation de concentrés comparatifs

Concentré 14 comparatif

Dans un flacon en verre de 60 mL, 40% en poids du mélange de MELs, 30% en poids de 2-octanol et 30% en poids de Radia® 7444 ont été ajoutés, les % en poids étant indiqués par rapport au poids total du concentré obtenu, puis agités manuellement jusqu'à homogénéisation du concentré. Lors de l'agitation, il est possible de chauffer à 40°C pour faciliter l'homogénéisation.

Concentré 15 comparatif

Dans un flacon en verre de 60 mL, 40% en poids du mélange de MELs, 30% en poids de 2-octanol et 30% en poids de Radia® 7402 ont été ajoutés, les % en poids étant indiqués par rapport au poids total du concentré obtenu, puis agités manuellement jusqu'à homogénéisation du concentré. Lors de l'agitation, il est possible de chauffer à 40°C pour faciliter l'homogénéisation.

Evaluation des propriétés tensioactives des concentrés 14 et 15

Un protocole de préparation d'émulsions à partir des concentrés 14 et 15 a été réalisé, conformément à l'étape (i) de la norme CIPAC MT 36.3 (« CIPAC method 2000. Prepared by the German Formulation Panel (DAPF). Chairman : G Menschel. »).

Dans un premier temps, les concentrés 14 et 15 sont mis en contact avec un liquide hydrophobe selon le protocole suivant :

- dans un flacon, 10% en poids du concentré 14 ou 15 et 90% en poids de Radia® 7955 ont été ajoutés, les % en poids étant indiqués par rapport au poids total de la composition obtenue, puis agités manuellement jusqu'à homogénéisation de la composition. Lors de l'agitation, il est possible de chauffer la composition à 40°C pour faciliter l'homogénéisation,

On obtient ainsi les compositions 14 et 15 comparatives.

Ces compositions 14 et 15 sont ensuite mises en contact avec de l'eau selon le protocole suivant :
Dans des éprouvettes graduées, 1% en poids des différentes compositions 14 et 15 a respectivement été ajouté à 99 % en poids d'eau standard.

Les éprouvettes graduées ont été refermées à l'aide d'un bouchon. Les éprouvettes graduées ont ensuite été retournées une fois.

Après 30 secondes, il a pu être observé à l'œil nu que des émulsions non-uniformes (incomplètes) se sont formées dans les éprouvettes : des gouttelettes étaient présentes dans chaque éprouvette.

Ces essais peuvent être comparé à l'essai de l'Exemple 2 réalisé avec le concentré 2 selon l'invention, comportant 40% en poids du mélange de MELS, 30% en poids de 2-octanol et 30% en poids de mono-oléate de polyéthylène glycol-600 de HLB = 13,2.

## Revendications

**1.** Concentré comprenant :

- au moins un lipide de mannosylérythritol, et
- au moins un ester d'acide gras et de polyéthylène glycol de HLB supérieur ou égal à 12,

dans lequel le ratio en poids MEL : ester d'acide gras et de polyéthylène glycol est compris entre 0,01 et 5.

**2.** Concentré selon la revendication 1, comprenant au moins un alcool ayant un nombre d'atomes de carbone compris entre 1 et 16.

**3.** Concentré selon l'une quelconque des revendications 1 ou 2, dans lequel l'ester d'acide gras et de polyéthylène glycol a un HLB compris entre 12 et 20.

4. Concentré selon l'une quelconque des revendications 1 à 3, dans lequel la quantité totale de MEL(s) est comprise entre 5 et 75% en poids, par rapport au poids total du concentré.

5. Concentré selon l'une quelconque des revendications 1 à 4, dans lequel la quantité totale d'ester(s) d'acide gras et de polyéthylène glycol est comprise entre 1 et 90% en poids, par rapport au poids total du concentré.

6. Concentré selon l'une quelconque des revendications 1 à 5, comprenant au moins deux MELs choisis parmi le groupe constitué par MEL-A, MEL-B, MEL-C et MEL-D.

7. Concentré selon l'une quelconque des revendications 1 à 6, comprenant en outre au moins un acide gras libre et/ou au moins un triglycéride.

8. Procédé de préparation d'un concentré selon l'une quelconque des revendications 1 à 7, comprenant une étape de mélangeage d'au moins un lipide de mannosylérythritol et d'au moins un ester d'acide gras et de polyéthylène glycol.

9. Composition comprenant un concentré selon l'une quelconque des revendications 1 à 7, et un liquide hydrophobe.

10. Procédé de préparation d'une composition selon la revendication 9, comprenant une étape de mélangeage d'un concentré selon l'une quelconque des revendications 1 à 7 avec un liquide hydrophobe.

11. Composition phytosanitaire comprenant un concentré selon l'une des revendications 1 à 7, ou une composition selon la revendication 9, et un principe actif pesticide.

12. Composition cosmétique comprenant un concentré selon l'une des revendications 1 à 7, ou une composition selon la revendication 9, et un principe actif cosmétique.

13. Emulsion comprenant une composition selon la revendication 9, une composition phytosanitaire selon la revendication 11, ou une composition cosmétique selon la revendication 12, et de l'eau.

14. Procédé de préparation d'une émulsion selon la revendication 13, comprenant une étape de mélangeage d'une composition selon la revendication 9, d'une composition phytosanitaire selon la revendication 11, ou d'une composition cosmétique selon la revendication 12, avec de l'eau.

15. Utilisation d'un concentré selon l'une quelconque des revendications 1 à 7, en tant qu'agent tensioactif.

16. Utilisation d'un concentré selon l'une quelconques des revendications 1 à 7, en tant qu'agent épaississant.

17. Utilisation d'un concentré selon l'une quelconques des revendications 1 à 7, ou d'une composition selon la revendication 9, en tant qu'adjuvant.

18. Utilisation selon la revendication 17, dans laquelle l'adjuvant possède une propriété mouillante.

## Patentansprüche

1. Konzentrat, umfassend:

   - mindestens ein Mannosylerythritol-Lipid, und
   - mindestens einen Polyethylenglykol-Fettsäureester mit einem HLB-Wert größer oder gleich 12,

   wobei das Verhältnis MEL : Polyethylenglykol-Fettsäureester zwischen 0,01 und 5 liegt.

2. Konzentrat nach Anspruch 1, umfassend mindestens einen Alkohol mit einer Anzahl von Kohlenstoffatomen, die zwischen 1 und 16 liegt.

3. Konzentrat nach einem der Ansprüche 1 oder 2, wobei der Polyethylenglykol-Fettsäureester einen HLB-Wert hat, der zwischen 12 und 20 liegt.

4. Konzentrat nach einem der Ansprüche 1 bis 3, wobei die Gesamtmenge von MEL(s) zwischen 5 und 75 Gew.-% in Bezug auf das Gesamtgewicht des Konzentrats liegt.

5. Konzentrat nach einem der Ansprüche 1 bis 4, wobei die Gesamtmenge von Polyethylenglykol-Fettsäureester(n) zwischen 1 und 90 Gew.-% in Bezug auf das Gesamtgewicht des Konzentrats liegt.

6. Konzentrat nach einem der Ansprüche 1 bis 5, umfassend mindestens zwei MELs, ausgewählt aus der Gruppe, die von MEL-A, MEL-B, MEL-C und MEL-D gebildet ist.

7. Konzentrat nach einem der Ansprüche 1 bis 6, umfassend ferner mindestens eine freie Fettsäure und/oder mindestens ein Triglycerid.

8. Verfahren zur Herstellung eines Konzentrats nach einem der Ansprüche 1 bis 7, umfassend einen Schritt des Mischens mindestens eines Mannosylerythritol-Lipids und mindestens eines Polyethylenglycol-Fettsäureesters.

9. Zusammensetzung, umfassend ein Konzentrat nach einem der Ansprüche 1 bis 7 und eine hydrophobe Flüssigkeit.

10. Verfahren zur Herstellung einer Zusammensetzung nach Anspruch 9, umfassend einen Schritt des Mischens eines Konzentrats nach einem der Ansprüche 1 bis 7 mit einer hydrophoben Flüssigkeit.

11. Pflanzenschutz-Zusammensetzung, umfassend ein Konzentrat nach einem der Ansprüche 1 bis 7 oder eine Zusammensetzung nach Anspruch 9 und ein Pestizid-Wirkprinzip.

12. Kosmetische Zusammensetzung, umfassend ein Konzentrat nach einem der Ansprüche 1 bis 7 oder eine Zusammensetzung nach Anspruch 9 und ein kosmetisches Wirkprinzip.

13. Emulsion, umfassend eine Zusammensetzung nach Anspruch 9, eine Pflanzenschutz-Zusammensetzung nach Anspruch 11 oder eine kosmetische Zusammensetzung nach Anspruch 12 und Wasser.

14. Verfahren zur Herstellung einer Emulsion nach Anspruch 13, umfassend einen Schritt des Mischens einer Zusammensetzung nach Anspruch 9, einer Pflanzenschutz-Zusammensetzung nach Anspruch 11 oder einer kosmetischen Zusammensetzung nach Anspruch 12 mit Wasser.

15. Verwendung eines Konzentrats nach einem der Ansprüche 1 bis 7 als Tensid.

16. Verwendung eines Konzentrats nach einem der Ansprüche 1 bis 7 als Verdickungsmittel.

17. Verwendung eines Konzentrats nach einem der Ansprüche 1 bis 7 oder einer Zusammensetzung nach Anspruch 9 als Adjuvans.

18. Verwendung nach Anspruch 17, wobei das Adjuvans eine benetzende Eigenschaft besitzt.

## Claims

1. Concentrate comprising:

   - at least one mannosylerythritol lipid, and
   - at least one polyethylene glycol fatty acid ester having an HLB value greater than or equal to 12,

   in which the MEL:polyethylene glycol fatty acid ester weight ratio is comprised between 0.01 and 5.

2. Concentrate according to claim 1, comprising at least one alcohol having a number of carbon atoms comprised between 1 and 16.

3. Concentrate according to any one of claims 1 or 2, in which the polyethylene glycol fatty acid ester has an HLB value comprised between 12 and 20.

**4.** Concentrate according to any one of claims 1 to 3, in which the total quantity of MEL(s) is comprised between 5 and 75% by weight, with respect to the total weight of the concentrate.

**5.** Concentrate according to any one of claims 1 to 4, in which the total quantity of polyethylene glycol fatty acid ester(s) is comprised between 1 and 90% by weight, with respect to the total weight of the concentrate.

**6.** Concentrate according to any one of claims 1 to 5, comprising at least two MELs chosen from the group constituted by MEL-A, MEL-B, MEL-C and MEL-D.

**7.** Concentrate according to any one of claims 1 to 6, also comprising at least one free fatty acid and/or at least one triglyceride.

**8.** Process for the preparation of a concentrate according to any one of claims 1 to 7, comprising a step of mixing at least one mannosylerythritol lipid and at least one polyethylene glycol fatty acid ester.

**9.** Composition comprising a concentrate according to any one of claims 1 to 7, and a hydrophobic liquid.

**10.** Process for the preparation of a composition according to claim 9, comprising a step of mixing a concentrate according to any one of claims 1 to 7 with a hydrophobic liquid.

**11.** Phytosanitary composition comprising a concentrate according to one of claims 1 to 7, or a composition according to claim 9, and a pesticide active ingredient.

**12.** Cosmetic composition comprising a concentrate according to one of claims 1 to 7, or a composition according to claim 9, and a cosmetic active ingredient.

**13.** Emulsion comprising a composition according to claim 9, a phytosanitary composition according to claim 11, or a cosmetic composition according to claim 12, and water.

**14.** Process for the preparation of an emulsion according to claim 13, comprising a step of mixing a composition according to claim 9, a phytosanitary composition according to claim 11, or a cosmetic composition according to claim 12, with water.

**15.** Use of a concentrate according to any one of claims 1 to 7, as surfactant.

**16.** Use of a concentrate according to any ones of the claims 1 to 7, as thickening agent.

**17.** Use of a concentrate according to any one of claims 1 to 7, or of a composition according to claim 9, as adjuvant.

**18.** Use according to claim 17, in which the adjuvant has a wetting property.

Fig. 1

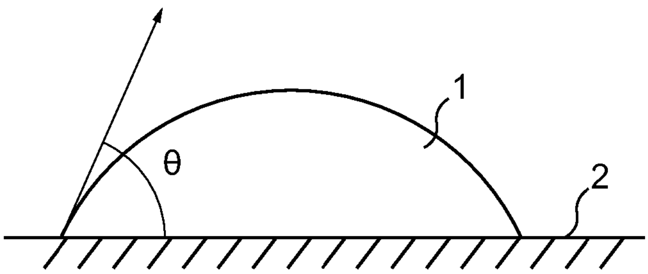


Fig. 2a

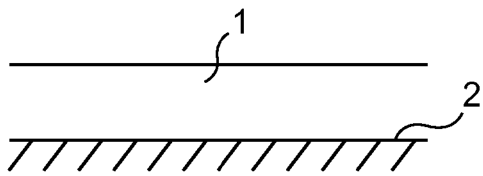


Fig. 2b

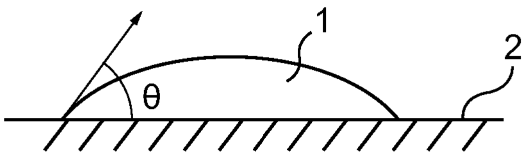


Fig. 2c

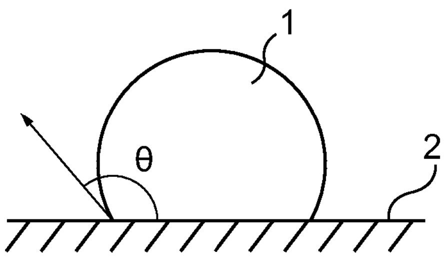

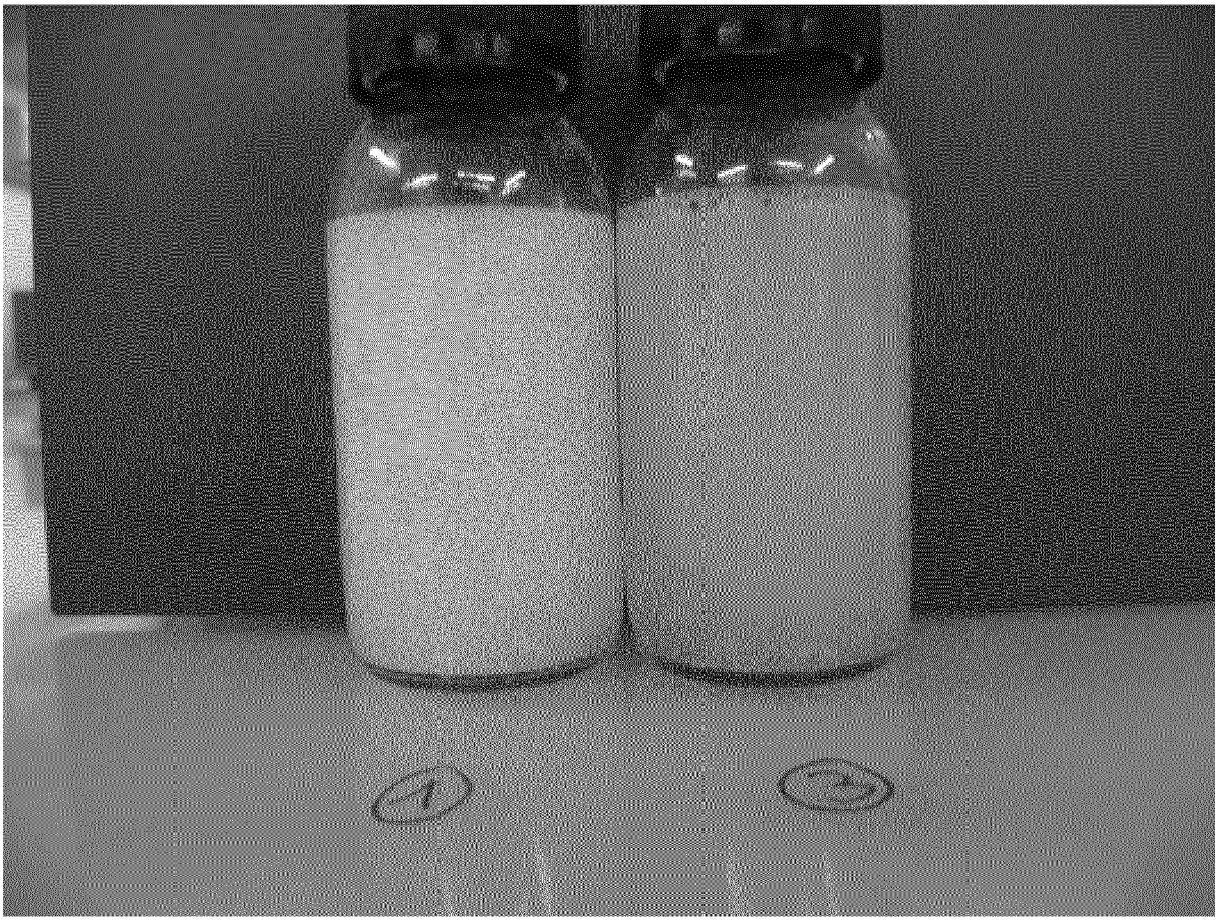


Fig. 3

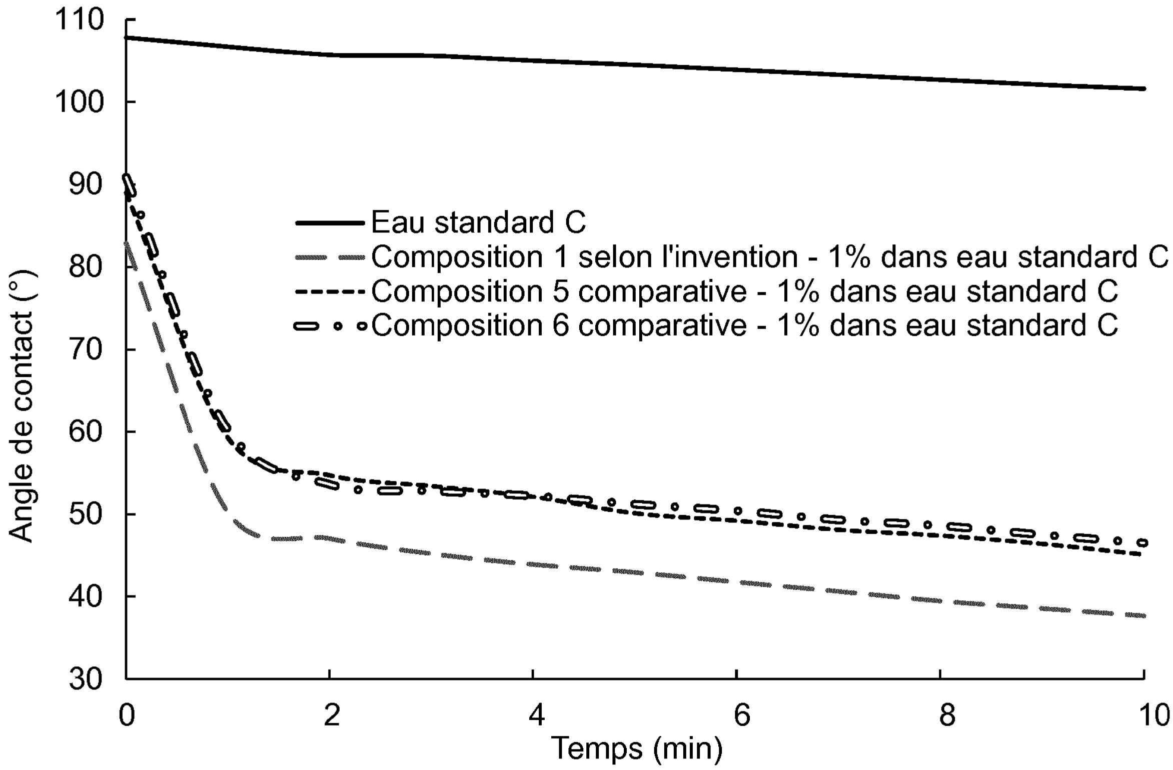
110
100
90
80
70
60
50
40
30
Angle de contact (°)
Eau standard C
Composition 1 selon l'invention - 1% dans eau standard C
Composition 5 comparative - 1% dans eau standard C
Composition 6 comparative - 1% dans eau standard C
0
2
4
6
8
10
Temps (min)

Fig. 4

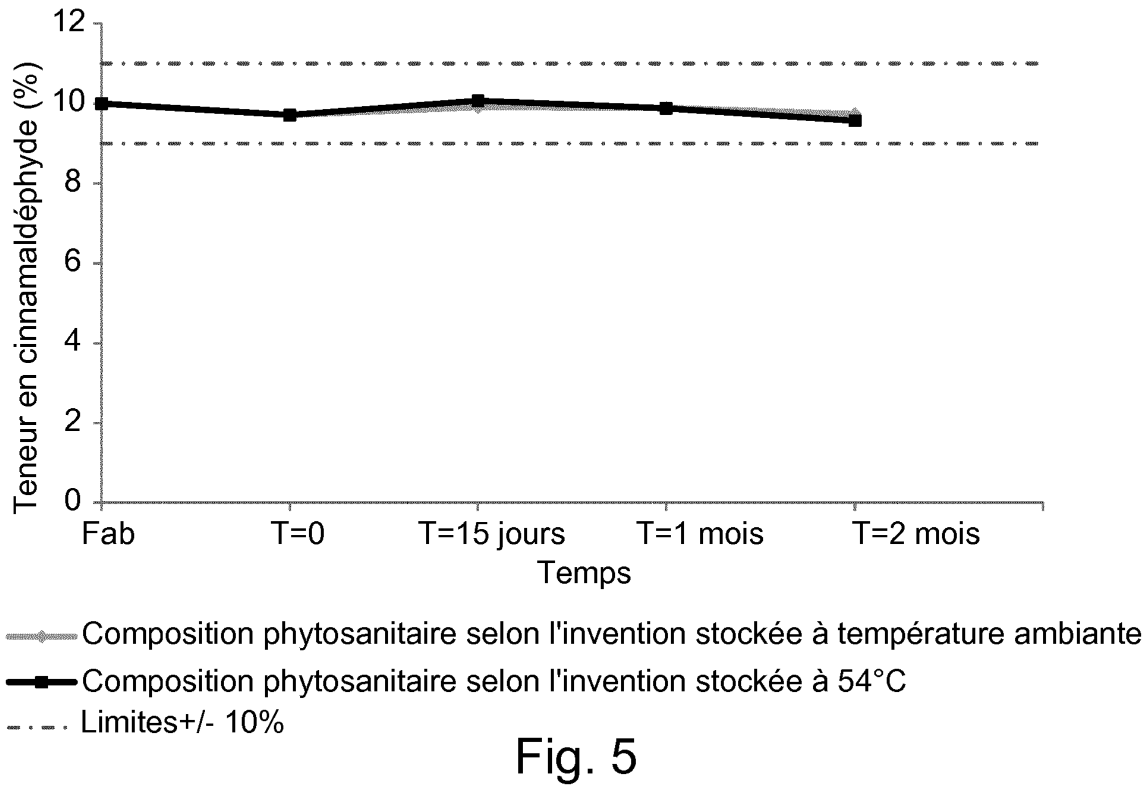
12
10
8
6
4
2
0
Teneur en cinnamaldéphyde (%)
Fab
T=0
T=15 jours
T=1 mois
T=2 mois
Temps
Composition phytosanitaire selon l'invention stockée à température ambiante
Composition phytosanitaire selon l'invention stockée à 54°C
Limites+/- 10%

Fig. 5

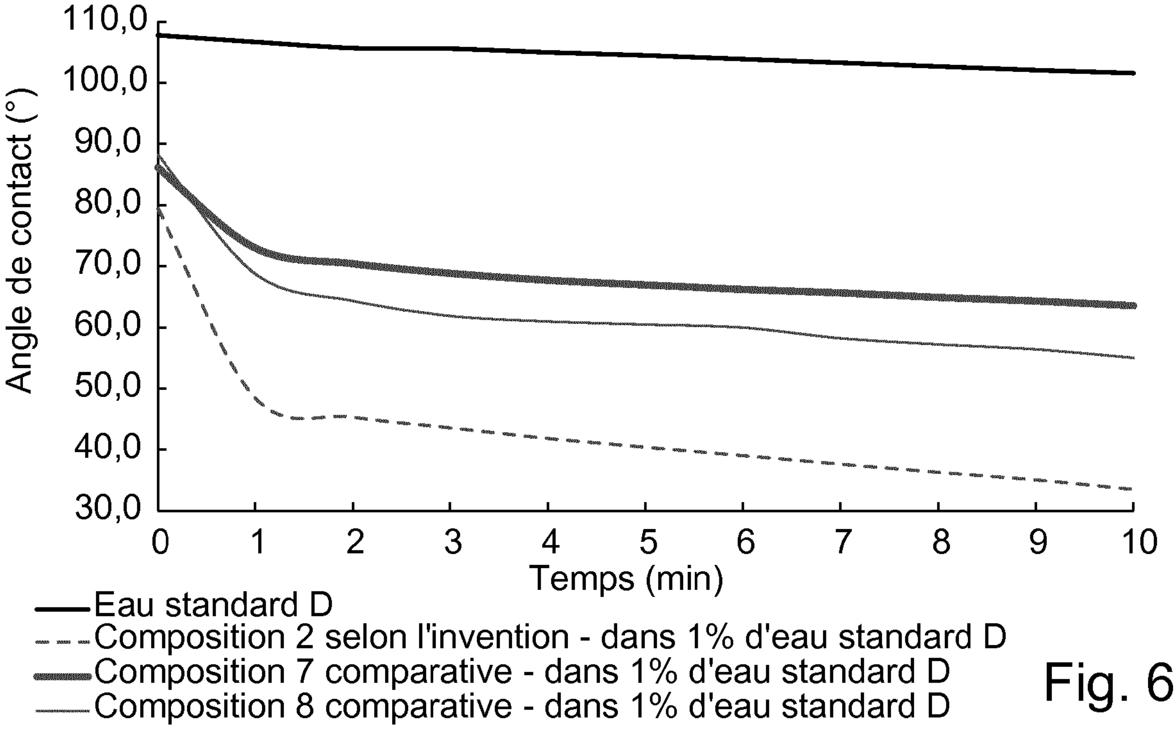
110,0
100,0
90,0
80,0
70,0
60,0
50,0
40,0
30,0
Angle de contact (°)
0
1
2
3
4
5
6
7
8
9
10
Temps (min)
Eau standard D
Composition 2 selon l'invention - dans 1% d'eau standard D
Composition 7 comparative - dans 1% d'eau standard D
Composition 8 comparative - dans 1% d'eau standard D

Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*


### Documents brevets cités dans la description

- EP 1964546 A1
- JP 2011026278 A
- JP 2009167158 A
- JP 2009167157 A
- JP 2010018560 A
- JP 2011026276 A
- US 2013142855 A1

### Littérature non-brevet citée dans la description

- **TOKUMA FUKUOKA et al.** Application of yeast glycolipid biosurfactant, mannosylerythritol lipid as agrospreaders. *Journal of oleo science,* 2015, vol. 64 (6), 689-695
- **KITAMOTO D. et al.** Microbial conversion of n-alkanes into glycolipid biosurfactants, mannosylerythritol lipids, by Pseudozyma (Candida Antartica). *Biotechnology Letters,* 2001, vol. 23 (20), 1709-1714
- **JOSEPH IRUDAYARAJ ARUTCHELVI et al.** Mannosylerythritol lipids: a review. *Journal of industrial microbiology & biotechnology,* 2008, vol. 35 (12), 1559-1570
- **MNIF INES et al.** Glycolipid biosurfactants: Potential related biomedical and biotechnological applications. *Carbohydrate Research,* 2015, vol. 416, 59-69
- **N. LOURITH et al.** Natural surfactants used in cosmetics: glycolipids. *International Journal of Cosmestic Science,* 2009, vol. 31 (4), 255-261
- **G MENSCHEL.** Prepared by the German Formulation Panel (DAPF). *CIPAC method 2000,* 2000
- **RAU et al.** Downstream processing of mannosylerythritol lipids produced by Pseudozyma aphidis. *European Journal of Lipids Science and Technology,* 2005, vol. 107, 373-380